# EUROPEAN PATENT APPLICATION

(11) **EP 1 475 372 A1**
(43) Date of publication of application: **10.11.2004**
(21) Application number: 03701142.6
(22) Date of filing: 21.01.2003
(51) Int. Cl.: C07D 265/38, C07D 279/36, C07D 513/04, C09K 11/06, H05B 33/14

(54) **NILE RED TYPE COMPOUND EMITTING RED LIGHT, PROCESS FOR PRODUCING THE SAME, AND LUMINESCENT ELEMENT UTILIZING THE SAME**

(30) Priority: 21.01.2002 JP 2002012224; 21.01.2002 JP 2002012222; 23.01.2002 JP 2002014881; 12.06.2002 JP 2002172127
(71) Applicant: Horose Engineering Co., Ltd., Tokyo 146-0092 (JP)
(72) Inventor: NAKAYA, Tadao c/o Horose Engineering Co., Ltd., Ohta-ku, Tokyo 146-0092 (JP); TAJIMA, Akio c/o Horose Engineering Co., Ltd., Ohta-ku, Tokyo 146-0092 (JP); SAIKAWA, Tomoyuki c/o Hirose Engineering Co.,Ltd., Ohta-ku, Tokyo 146-0092 (JP); TAKANO, Shinji c/o Hirose Engineering Co., Ltd., Ohta-ku, Tokyo 146-0092 (JP); YAMAUCHI, Takao, Yokohama-shi, Kanagawa 230-0074 (JP); MORI, Hidemasa, Yokohama-shi, Kanagawa 246-0034 (JP)
(74) Representative: Olgemöller, Luitgard, Dr.
(86) International application number: PCT/JP2003/000477
(87) International publication number: WO 2003/062213

(57) **Abstract**

The objectives of this invention are to provide a novel Nile red luminescent compound capable of emitting red light at a high luminance and at high color purity, and a luminescence element capable of emitting light at a high luminance. To achieve these obj ectives, the present invention provides a Nile red luminescent compound emitting red light represented by formula (1) and a luminescence element that includes the compound in the light-emitting layer thereof.

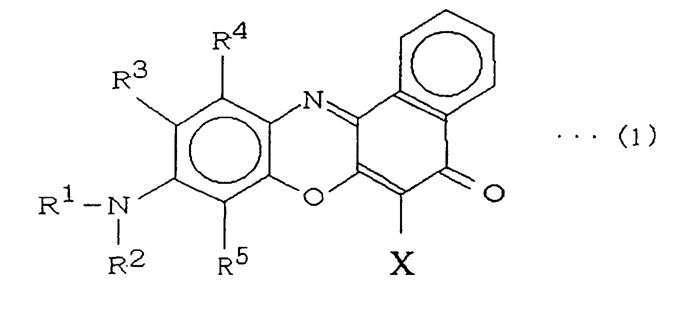

## Description

### Technical Field

The present invention relates to a Nile red luminescent compound emitting red light, a process for producing the same and a luminescence element utilizing the same. More particularly, this invention relates to a Nile red luminescent compound capable of emitting at a high luminance a light of which color is nearly crimson upon the application of electric energy, a novel process of producing the compound and a luminescence element utilizing the same.

### Background Art

For organic electroluminescence elements, which are often abbreviated to "organic EL elements", have been proposed various organic compounds.

However, compounds that are capable of emitting red light at a high luminance and endurable against heat, light, etc. have not been developed.

The objective of this invention is to provide an organic compound capable of emitting red light at a high luminance, and/or capable of emitting a light of which color is such a red that the value on the x-axis in the CIE chromaticity is over 0.62, particularly 0.63, and further endurable against heat, light, etc. This invention also aims for providing a process for producing the organic compound and a luminescence element utilizing the compound.

### Summary of the Invention

In order to solve the aforementioned problems, this invention provides a Nile red luminescent compound emitting red light that has a structure represented by formula (1): 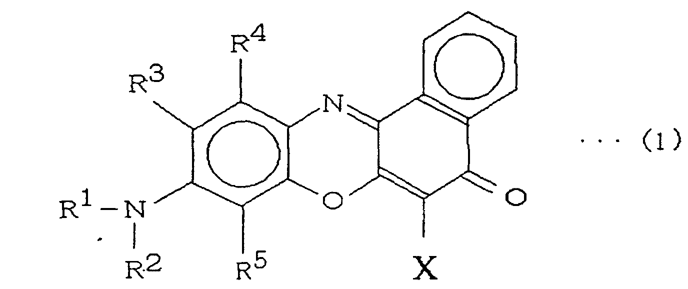 wherein R¹ is hydrogen atom or an alkyl group, or forms -CH₂CH₂-CR⁶R⁷- together with R³ (wherein the carbon atom of -CR⁶R⁷- moiety is bound to the benzene moiety of chemical formula (1), each of R⁶ and R⁷ is hydrogen atom or an alkyl group, and R⁶ and R⁷ may be the same or different from each other); R² is hydrogen atom or an alkyl group, or forms -CH₂CH₂-CR⁸R⁹- together with R⁵ (wherein the carbon atom of -CR⁸R⁹- moiety is bound to the benzene moiety of chemical formula (1), each of R⁸ and R⁹ is hydrogen atom or an alkyl group, and R⁸ and R⁹ may be the same or different from each other); R³ is hydrogen atom, forms -CH₂CH₂-CR⁶R⁷- with R¹, or forms with R⁴ a naphthalene ring including as a part thereof the benzene moiety of chemical formula (1); R⁴ is hydrogen atom, or forms with R³ a naphthalene ring including as a part thereof the benzene moiety of chemical formula (1); R⁵ is hydrogen atom, or forms -CH₂CH₂-CR⁸R⁹- with R²; and X is a halogen atom.

The process for producing the Nile red luminescent compound emitting red light represented by formula (1) is characterized by reacting a Nile red pigment compound represented by formula (2) with a halogenating agent. 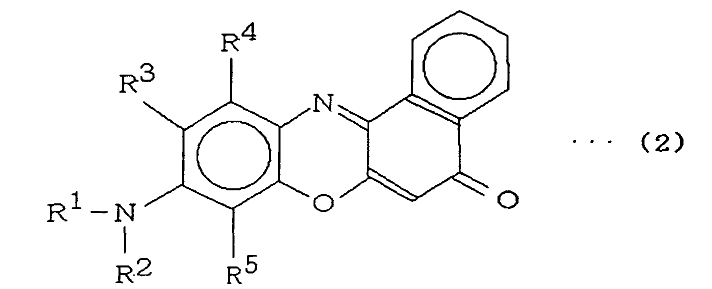 In the formula, R¹, R², R³, R⁴ and R⁵ mean the same atoms and groups as those defined above.

The present invention also provides a Nile red compound emitting red light having the structure represented by formula (3). 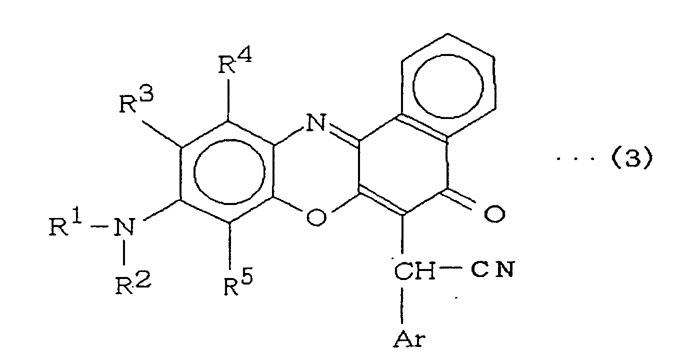 In the formula, R¹, R², R³, R⁴ and R⁵ mean the same atoms and groups as those defined above. Ar means one of formulae (4), (6) and (7) : 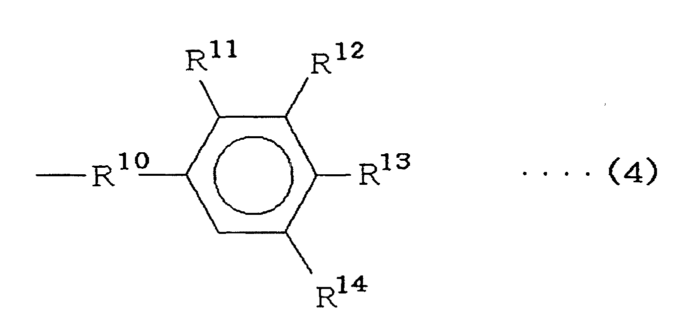 wherein R¹⁰ is a single chemical bond or methylene group; R¹¹ is hydrogen atom, or forms -CF₂-O-CF₂- with R¹²; R¹² is fluorine atom, cyano group or a lower alkyl having 1-5 carbon atoms and at least one fluorine atom, forms -CF₂-O-CF₂- with R¹¹, or forms -CF₂-O-CF₂- with R¹³; R¹³ is hydrogen atom, cyano group, fluorine atom or a lower alkyl having 1-5 carbon atoms and at least one fluorine atom, forms -CF₂-O-CF₂- with R¹², or is a group represented by formula (5); and R¹⁴ is hydrogen atom or a lower alkyl having 1-5 carbon atoms and at least one fluorine atom when R¹³ is hydrogen atom, and R¹⁴ is hydrogen atom when R¹³ is not hydrogen atom, 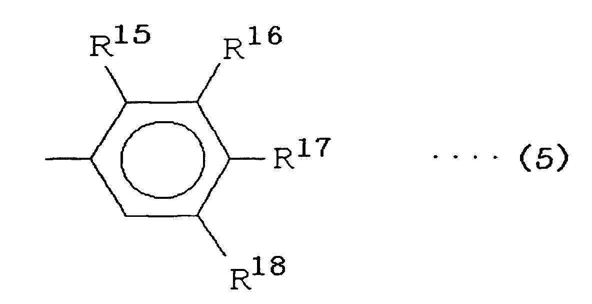 wherein R¹⁵ is hydrogen atom, or forms -CF₂-O-CF₂- with R¹⁶; R¹⁶ is fluorine atom, cyano group or a lower alkyl having 1-5 carbon atoms and at least one fluorine atom, forms -CF₂-O-CF₂- with R¹⁵, or forms -CF₂-O-CF₂- with R¹⁷; R¹⁷ is hydrogen atom, cyano group, fluorine atom or a lower alkyl having 1-5 carbon atoms and at least one fluorine atom, or forms -CF₂-O-CF₂- with R¹⁶; and R¹⁸ is hydrogen atom or a lower alkyl having 1-5 carbon atoms and at least one fluorine atom when R¹⁷ is hydrogen atom, and R¹⁸ is hydrogen atom when R¹⁷ is not hydrogen atom, 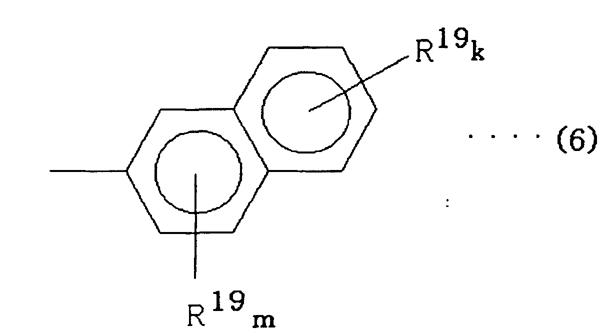 wherein R¹⁹ is fluorine atom, cyano group, or a lower alkyl having 1-5 carbon atoms and at least one fluorine atom; k is an integer of 1-4, m is an integer of 1-3, and all of the R¹⁹ groups may be the same or different from each other, 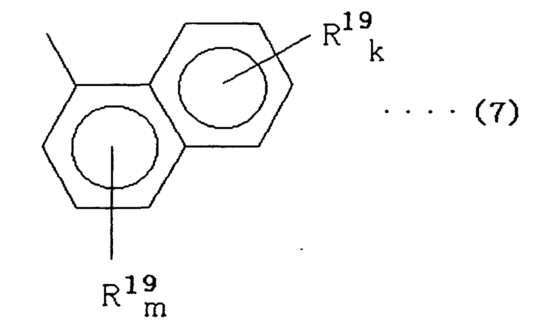 wherein R¹⁹ is fluorine atom, cyano group, or a lower alkyl having 1-5 carbon atoms and at least one fluorine atom; k is an integer of 1-4, m is an integer of 1-3, and all of the R¹⁹ groups may be the same or different from each other.

The process of preparing a Nile red luminescent compound emitting red light represented by formula (3) comprises reacting a Nile red pigment compound represented by formula (2) with an electron attractive aromatic acetonitrile represented by formula (8): 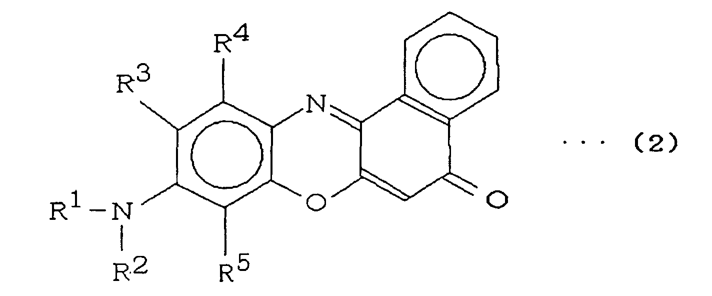 wherein R¹, R², R³, R⁴ and R⁵ are respectively the same as those defined in the explanation of formula (3),

NC-CH₂-Ar (8)

wherein Ar is the same as that defined in the explanation of formula (3).

The present invention further provides a Nile red luminescent compound emitting red light that has the structure represented by formula (9): 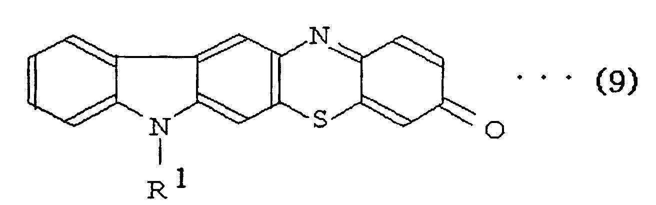 wherein R¹ is hydrogen atom or an alkyl group.

The present invention still further provides a Nile red luminescent compound emitting red light that has the structure represented by formula (10): 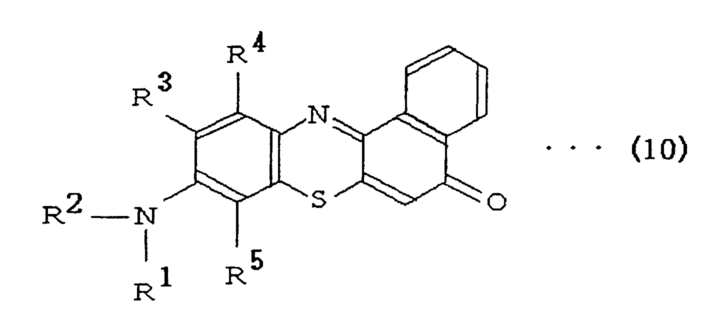 wherein R¹, R², R³, R⁴ and R⁵ are respectively the same as those defined in the explanation of formula (1).

The process of producing a Nile red luminescent compound represented by formula (9) comprises reacting 4-nitrosophenol with a carbazole, the nitrogen atom of which is bonded with a substituent R¹, wherein R¹ is hydrogen atom or an alkyl group, and reacting the compound obtained in the previous reaction with sulfur.

The process of producing a Nile red luminescent compound represented by formula (10) comprises reacting 1-naphphol with a 4-nitrosoaniline, the amino group of which is bonded with substituents R¹ and R², wherein each of R¹ and R² is hydrogen atom or an alkyl group, and R¹ and R² may be the same or different from each other; and reacting the compound obtained in the previous reaction with sulfur.

The present invention provides another Nile red luminescent compound emitting red light that has the structure represented by formula (11) : 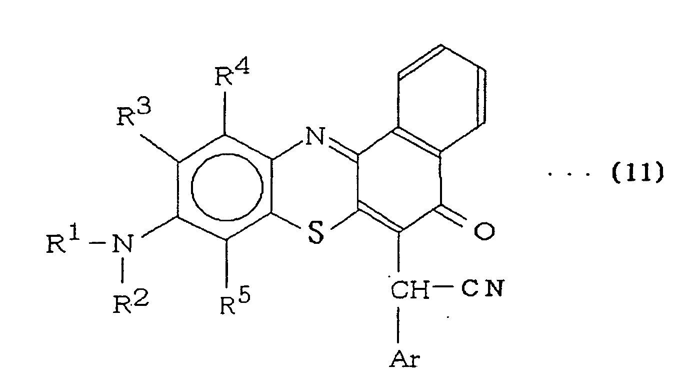 wherein R¹, R², R³, R⁴ and R⁵ are respectively the same as those defined in the explanation of formula (1), and Ar is also the same as that defined in the explanation of formula (3).

The process of producing a Nile red luminescent compound emitting red light represented by formula (11) comprises reacting a Nile red luminescent compound emitting red light represented by formula (10) with an electron attractive aromatic acetonitrile represented by the formula NC-CH₂-Ar, wherein Ar is the same as that defined in the explanation of formula (3).

The present invention also provides a luminescence element comprising a pair of electrodes and a light-emitting layer including at least one of the Nile red luminescent compounds represented by formulas (1), (3), (9), (10) and (11) between the electrodes.

In a preferred embodiment of the invention relating to the luminescence element, the element further comprises a hole-transporting layer between the light-emitting layer and the cathode, which is one of the electrodes.

In another preferred embodiment of this invention relating to the luminescence element, the light-emitting layer includes at least one of the Nile red luminescent compounds represented by formulas (1), (3), (9), (10) and (11), and a host pigment.

In a further preferred embodiment of this invention relating to the luminescence element, the light-emitting layer and the hole-transporting layer are formed by vapor deposition.

In a still further embodiment of this invention relating to the luminescence element, the light-emitting layer includes at least one of the Nile red luminescent compounds represented by formulas (1), (3), (9), (10) and (11), an electron-transporting substance, and a hole-transporting high polymer.

In another preferred embodiment of this invention, the light-emitting layer is formed through the application of the layer.

### Brief Description of the Drawings

Figure 1 is an illustration showing an example of the luminescence element in accordance with the present invention.
Figure 2 is an illustration showing another example of the luminescence element in accordance with the present invention.
Figure 3 is an illustration showing a still another example of the luminescence element in accordance with the present invention.
Figure 4 is an illustration showing a further example of the luminescence element in accordance with the present invention.
Figure 5 is an IR chart of the Nile red luminescent compound synthesized in Example 1 as an example of the present invention.
Figure 6 is an NMR chart of the Nile red luminescent compound synthesized in Example 1.
Figure 7 is a fluorescence spectrum of the Nile red luminescent compound synthesized in Example 1.
Figure 8 is an IR chart of the Nile red luminescent compound synthesized in Example 2 as an example of the present invention.
Figure 9 is an NMR chart of the Nile red luminescent compound synthesized in Example 2.
Figure 10 is a fluorescence spectrum of the Nile red luminescent compound synthesized in Example 2.
Figure 11 is a ¹H-NMR chart of Nile red luminescent compound A in Example 3.
Figure 12 is an IR chart of Nile red luminescent compound A in Example 3.
Figure 13 is an IR chart of Nile red luminescent compound B in Example 4.
Figure 14 is an NMR chart of the Nile red luminescent compound in Example 18.
Figure 15 is an NMR chart of the Nile red luminescent compound in Example 19.
Figure 16 is an NMR chart of the Nile red luminescent compound in Example 12.
Figure 17 is an NMR chart of the Nile red luminescent compound in Example 20.
Figure 18 is an NMR chart of the Nile red luminescent compound in Example 21.
Figure 19 is an NMR chart of 6-amino-3-(diisopropylamino)-2-nitrophenol in Example 22.
Figure 20 is an NMR chart of the Nile red compound derivative in Example 22.
Figure 21 is an NMR chart of the Nile red luminescent compound in Example 22.
Figure 22 is an NMR chart of the intermediate (a1) prepared during Example 23.
Figure 23 is an NMR chart of the Nile red luminescent compound emitting red light (b1) prepared in Example 23.
Figure 24 is an NMR chart of the intermediate (c1) prepared during Example 24.
Figure 25 is an NMR chart of the Nile red luminescent compound emitting red light (d1) prepared in Example 24.
Figure 26 is an NMR chart of the Nile red luminescent compound emitting red light, which is represented by formula (10), prepared in Example 25.
Figure 27 is an IR chart of the Nile red luminescent compound emitting red light, which is represented by formula (10), prepared in Example 25.
Figure 28 is a fluorescence spectrum of the Nile red luminescent compound emitting red light, which is represented by formula (10), prepared in Example 25.
Figure 29 is an IR chart of the Nile red luminescent compound emitting red light, which is represented by formula (18), prepared in Example 26.
Figure 30 is an NMR chart of the Nile red luminescent compound emitting red light, which is represented by formula (18), prepared in Example 26.
Figure 31 is a fluorescence spectrum of the Nile red luminescent compound emitting red light, which is represented by formula (18), prepared in Example 26.

### Detailed Description of the Preferred Embodiments

The Nile red luminescent compound in accordance with this invention is represented by formula (1):

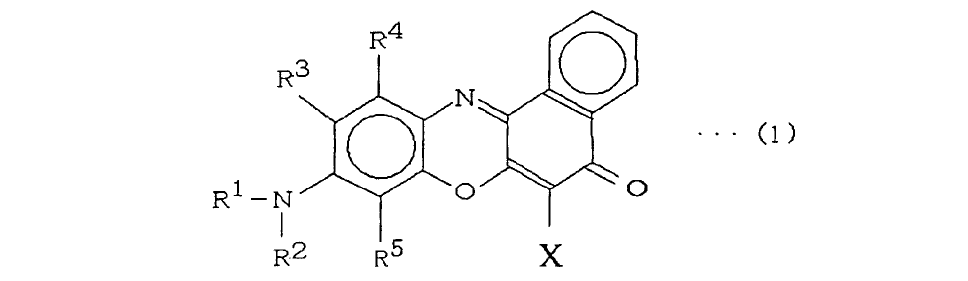

In this formula, R¹ is hydrogen atom or an alkyl group, preferably a lower alkyl group having 1-5 carbon atoms. The lower alkyl group includes methyl group, ethyl group, propyl group, butyl group and pentyl group.

R² is hydrogen atom or an alkyl group, preferably a lower alkyl group having 1-5 carbon atoms, which includes the same groups as R¹. R¹ and R² may be the same lower alkyl group or different from each other.

Together with R³, R¹ forms -CH₂CH₂-CR⁶R⁷- (wherein the carbon atom of -CR⁶R⁷- moiety is bound to the benzene moiety of chemical formula (1), each of R⁶ and R⁷ is hydrogen atom or a lower alkyl having 1-5 carbon atoms, and R⁶ and R⁷ may be the same or different from each other).

When R¹ and R² are lower alkyl groups, preferable -NR¹R² includes diethylamino group, di-n-propylamino group, di-i-propylamino group, butyl group, etc.

Together with R⁵, R² forms -CH₂CH₂-CR⁸R⁹- (wherein the carbon atom of -CR⁸R⁹- moiety is bound to the benzene moiety of chemical formula (1), each of R⁸ and R⁹ is hydrogen atom or a lower alkyl having 1-5 carbon atoms, and R⁸ and R⁹ may be the same or different from each other).

When R¹ forms -CH₂CH₂-CR⁶R⁷- with R³, and R² forms -CH₂CH₂-CR⁸R⁹- with R⁵, formula (1) becomes the following formula (12) :

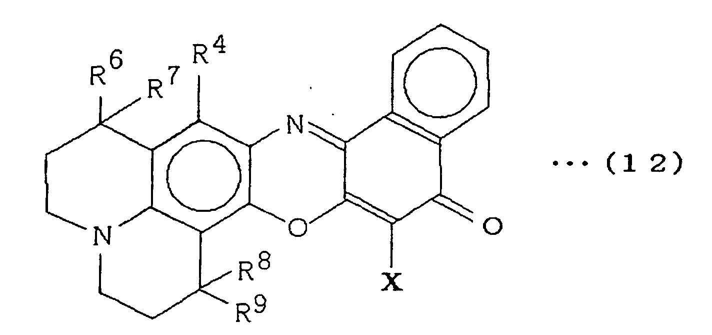

In this formula (12), R⁴, R⁶, R⁷, R⁸, R⁹ and X denote the same as those mentioned above.

Both R³ and R⁴ may be hydrogen atoms, or form together a naphthalene ring including as a part thereof the benzene moiety of chemical formula (1). The red light-emitting luminescent compound that has the naphthalene ring including as a part thereof the benzene moiety formed by R³ and R⁴ is represented by formula (13).

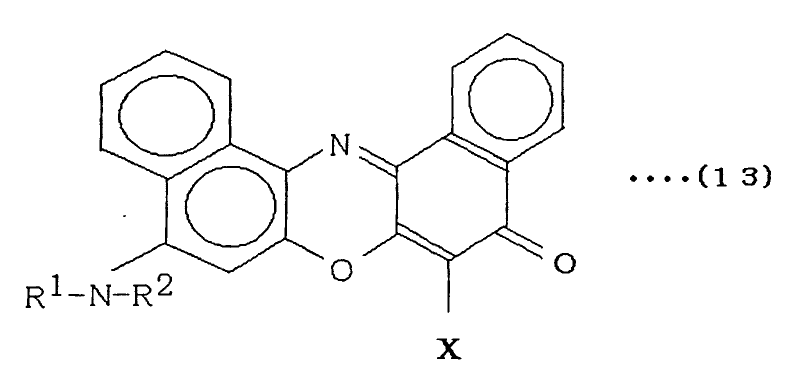

In formula (13), R¹, R² and X denote the same as those mentioned above.

In formula (1), X may be fluorine atom, chlorine atom, bromine atom, or iodine atom.

In the Nile red luminescent compound represented by formula (1), -NR¹R² is an electron-donating group and the halogen atom represented by X is an electron attractive group, so that π electron cloud on the skeleton of the Nile red compound is extended to the substituents. Therefore, we suppose that the application of a little energy enables the luminescent compound to emit red light. The novel luminescent compound of this invention is characterized by a structure where R¹-N-R², the electron-donating group, provides the π electron cloud with electrons. Because this Nile red skeleton has an electronically stable structure and therefore the Nile red luminescent compound is chemically stable, the luminescent compound does not deteriorate even under severe environments, which is a special character of the compound.

A Nile red luminescent compound emitting red light represented by formula (1) may be prepared by the following method.

The compound may be obtained by reacting a Nile red compound represented by general formula (2) with a halogenating agent.

The halogenating agent may be a common one that is able to replace hydrogen atoms on an aromatic ring with halogen atoms. Specific examples of the halogenating agent are sulfuryl chloride, phosphorus pentachloride, etc. when hydrogen atoms on an aromatic ring are replaced with chlorine atoms. Generally, when hydrogen atoms on an aromatic ring are replaced with halogen atoms, an imido-N-halosuccinate such as an imido-N-bromosuccinate, and a dialkyl halomalonate such as a dialkyl bromomalonate may be used.

The Nile red compound represented by formula (2) and the halogenating agent react easily by heating them in a solvent. The solvent includes acetic anhydride, acetic acid, an acid anhydride having not more than 5 carbon atoms, an aromatic solvent such as benzene or toluene, a dioxane, etc. The reaction temperature usually ranges between 100 and 250°C, preferably between 100 and 170°C. After the reaction, purification and separation by an ordinary method will provide the aimed Nile red luminescent compound emitting red light.

The Nile red luminescent compound in accordance with this invention can easily be produced only by heating a mixture of the Nile red compound and the halogenating agent. A simple production method such as the above-mentioned is an industrial method.

Another Nile red luminescent compound emitting red light is represented by formula (3).

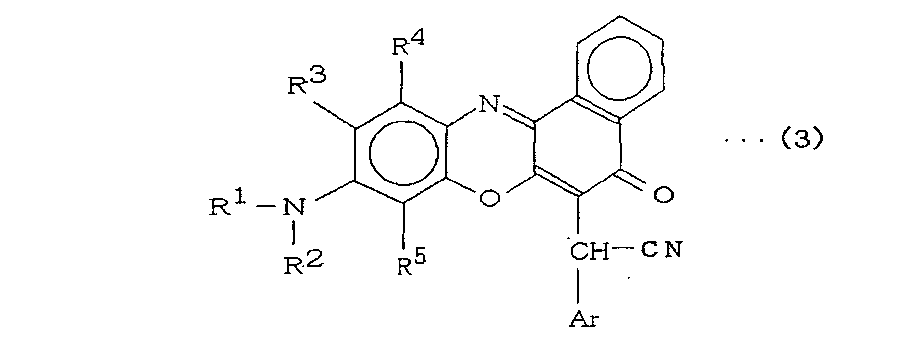

In formula (3), R¹ is hydrogen atom or an alkyl group, for example, a lower alkyl group having 1-5 carbon atoms. The lower alkyl group includes methyl group, ethyl group, propyl group, butyl group and pentyl group.

R² is hydrogen atom or an alkyl group, for example, a lower alkyl group having 1-5 carbon atoms, which includes the same groups as R¹. R¹ and R² may be the same lower alkyl group or different from each other.

Together with R³, R¹ forms -CH₂CH₂-CR⁶R⁷- (wherein the carbon atom of -CR⁶R⁷- moiety is bound to the benzene moiety of formula (3), each of R⁶ and R⁷ is hydrogen atom or a lower alkyl having 1-5 carbon atoms, and R⁶ and R⁷ may be the same or different from each other).

When R¹ and R² are lower alkyl groups, preferable -NR¹R² includes diethylamino group, di-n-propylamino group, di-i-propylamino group, butyl group, etc.

Together with R⁵, R² forms -CH₂CH₂-CR⁸R⁹- (wherein the carbon atom of -CR⁸R⁹- moiety is bound to the benzene moiety of formula (3), each of R⁸ and R⁹ is hydrogen atom or a lower alkyl having 1-5 carbon atoms, and R⁸ and R⁹ may be the same or different from each other).

When R¹ forms -CH₂CH₂-CR⁶R⁷- with R³, and R² forms -CH₂CH₂-CR⁸R⁹- with R⁵, formula (3) becomes the following formula (14):

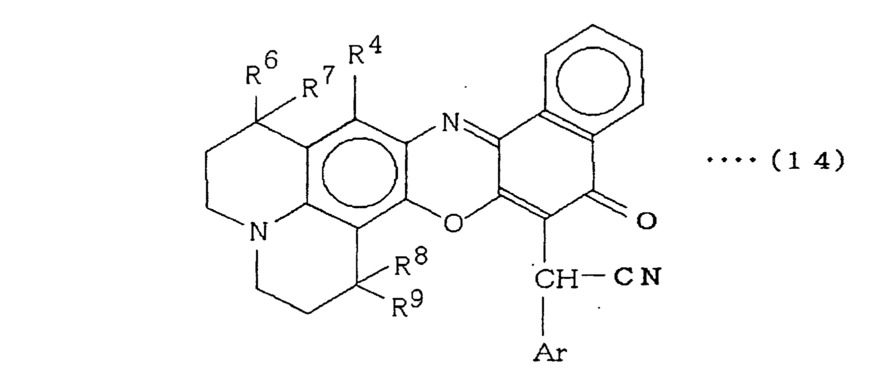

In this formula (12), R⁴, R⁶, R⁷, R⁸, and R⁹ denote the same as those mentioned above. Ar will be explained hereinafter.

Both R³ and R⁴ may be hydrogen atoms, or form together a naphthalene ring including as a part thereof the benzene moiety of formula (3). The red light-emitting luminescent compound that has the naphthalene ring including as a part thereof the benzene moiety formed by R³ and R⁴ is represented by formula (15).

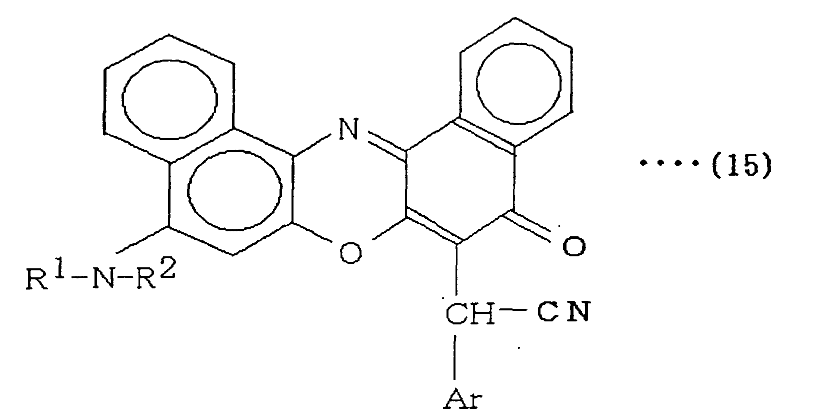

In formula (15), R¹ and R² denote the same as those mentioned above.

In formula (3), Ar has a structure represented by formula (4), (6) or (7).

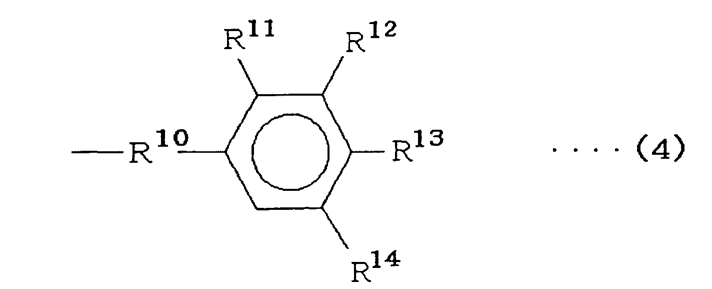

In this formula, R¹⁰ is a single chemical bond or methylene group.

R¹¹ is hydrogen atom, or forms -CF₂-O-CF₂- with R¹².

R¹² is fluorine atom, cyano group or a lower alkyl group having 1-5 carbon atoms and at least one fluorine atom, forms -CF₂-O-CF₂- with R¹¹, or forms -CF₂-O-CF₂- with R¹³. In this context, "a lower alkyl group having at least one fluorine atom" means a lower alkyl group, at least one hydrogen atom of which is replaced with a fluorine atom.

R¹³ is hydrogen atom, cyano group, fluorine atom or a lower alkyl having 1-5 carbon atoms and at least one fluorine atom, forms -CF₂-O-CF₂- with R¹², or is a group represented by formula (5).

R¹⁴ is hydrogen atom or a lower alkyl that has 1-5 carbon atoms and at least one fluorine atom when R¹³ is hydrogen atom, and R¹⁴ is hydrogen atom when R¹³ is not hydrogen atom.

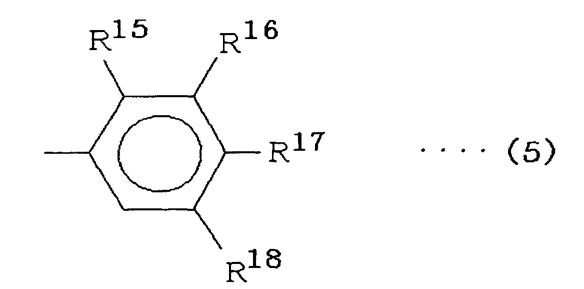

In this formula, R¹⁵ is hydrogen atom, or forms -CF₂-O-CF₂- with R¹⁶.

R¹⁶ is fluorine atom, cyano group or a lower alkyl group having 1-5 carbon atoms and at least one fluorine atom, forms -CF₂-O-CF₂- with R¹⁵, or forms -CF₂-O-CF₂- with R¹⁷.

R¹⁷ is hydrogen atom, cyano group, fluorine atom or a lower alkyl group having 1-5 carbon atoms and at least one fluorine atom, or forms -CF₂-O-CF₂- with R¹⁶.

R¹⁸ is hydrogen atom when R¹⁷ is not hydrogen atom, or a lower alkyl having 1-5 carbon atoms and at least one fluorine atom when R¹⁷ is hydrogen atom.

The lower alkyl group having 1-5 carbon atoms and at least one fluorine atom represented by R¹², R¹³, R¹⁶ and R¹⁷ includes a methyl group including at least one fluorine atom such as trifluoromethyl group, difluoromethyl group and monofluoromethyl group, an ethyl group including at least one fluorine atom such as pentafluoroethyl group, a propyl group including at least one fluorine atom such as hexafluoropropyl group, a pentyl group including at least one fluorine atom, etc. Among them, preferable is the methyl group including at least one fluorine atom.

In Ar of formula (3), (14) or (15), when R¹⁰ is a single chemical bond or methylene group, examples of the preferable combination of R¹¹, R¹², R¹³ and R¹⁴ are shown in Table 1.

**Table 1**

| Combination Examples | R¹¹ | R¹² | R¹³ | R¹⁴ |
|---|---|---|---|---|
| 1 | -H | -CF₃ | -H | -CF₃ |
| 2 | -H | -F | -CF₃ | -H |
| 3 | -H | -CF₃ | -F | -H |
| 4 | -H | -CF₃ | -CN | -H |
| 5 | -H | -CN | -CF₃ | -H |
| 6 | -H | -F | -CN | -H |
| 7 | -H | -CN | -F | -H |
| 8 | -H | -F | -CF₃ | -H |
| 9 | -CF₂-O-CF₂- | | -H | -H |
| 10 | -H | -CF₂-O-CF₂- | | -H |
| 11 | -H | General formula (5) | -H, -CF₃, -CN or -H | -H |
| 12 | -F | -F | -CF₃ | -H |
| 13 | -CF₃ | -H | -CF₃ | -H |

Other than the preferable examples shown in Table 1, also preferred examples of Ar are as follows: a fluorinated phenyl group such as 2,4-difluorophenyl group, 2,5-difluorophenyl group, 2,6-difluorophenyl group, 3,4-difluorophenyl group, 3, 5-difluorophenyl group, etc.; a trifluoromethylphenyl group such as 3-trifluoromethylphenyl group, 4-trifluoromethylphenyl group, 3, 5-bis (trifluoromethyl) phenyl group, etc.; and a fluorotrifluoromethylphenyl group such as 4-fluoro-3-trifluoromethylpheyl group, 6-fluoro-2-trifluoromethylphenyl group, etc.

Ar is also shown by formula (6): 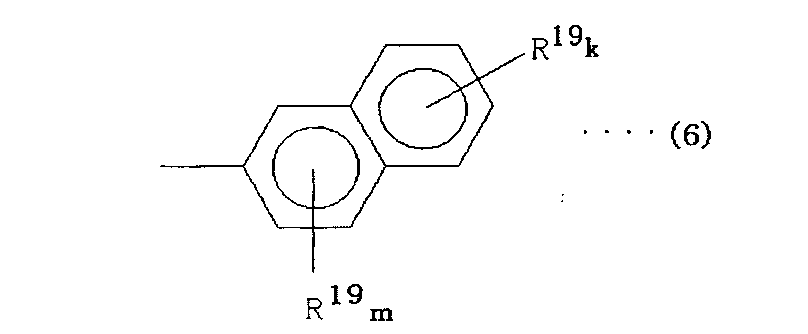 wherein R¹⁹ is fluorine atom, cyano group, or a lower alkyl group having 1-5 carbon atoms and at least one fluorine atom; k is an integer of 1-4, m is an integer of 1-3, and all of the R¹⁹ groups may be the same or different from each other.

Ar is further represented by formula (7): 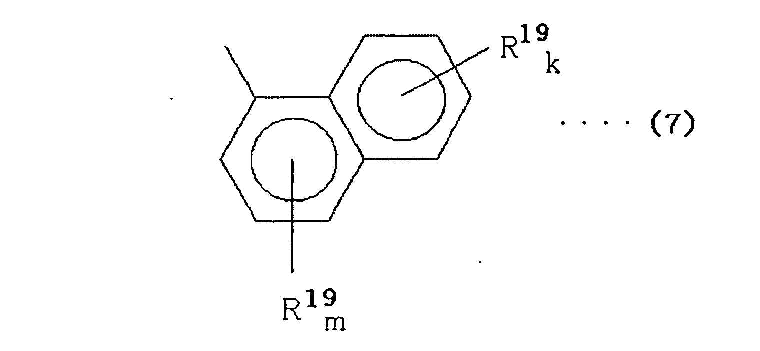 wherein R¹⁹ is fluorine atom, cyano group, or a lower alkyl group having 1-5 carbon atoms and at least one fluorine atom; k is an integer of 1-4, m is an integer of 1-3, and all of the R¹⁹ groups may be the same or different from each other.

Because the naphthyl group represented by formula (6) or (7) has electron attractive groups such as fluorine atom, cyano group and a lower alkyl group having 1-5 carbon atoms and at least one fluorine atom, π electrons on the skeleton of the Nile red compound and the fluorinated substituents or the cyano groups are super-conjugated, which facilitates the emission of red light.

The lower alkyl group having 1-5 carbon atoms and at least one fluorine atom, bonded to the naphthyl group, is the same as that in formula (4). Among them, trifluoromethyl group is preferable.

Among the naphthyl groups represented by formula (6), 1-naphthyl groups include, for example: a (trifluoromethyl)-1-naphthyl group having a trifluoromethyl group at the 2-, 3-, 4-, 5-, 6-, 7- or 8-position; a fluoro-1-naphthyl group having a fluorine atom at the 2-,3-,4-,5-, 6-, 7- or 8-position; a bis(trifluoromethyl)-1-naphthyl group having two trifluoromethyl groups at the 2- and 3-positions, the 2- and 4-positions, the 2- and 5-positions, the 2- and 6-positions, the 2- and 7-positions, the 2- and 8-positions, the 3- and 4-positions, the 3- and 5-positions, the 3- and 6-positions, the 3- and 7-positions, the 3- and 8-positions, the 4- and 5-positions, the 4- and 6-positions, the 4- and 7-positions, the 4- and 8-positions, the 5- and 6-positions, the 5- and 7-positions, the 5- and 8-positions, the 6- and 7-positions, the 6- and 8-positions, or the 7- and 8-positions; a difluoro-1-naphthyl group having two fluorine atoms at the 2-and 3-positions, the 2- and 4-positions, the 2- and 5-positions, the 2- and 6-positions, the 2- and 7-positions, the 2- and 8-positions, the 3- and 4-positions, the 3- and 5-positions, the 3- and 6-positions, the 3- and 7-positions, the 3- and 8-positions, the 4- and 5-positions, the 4- and 6-positions, the 4- and 7-positions, the 4- and 8-positions, the 5- and 6-positions, the 5- and 7-positions, the 5- and 8-positions, the 6- and 7-positions, the 6- and 8-positions, or the 7- and 8-positions; a tri(trifluoromethyl)-1-naphthyl group having three trifluoromethyl groups at the 2-, 3- and 4-positions, the 2-, 3- and 5-positions, the 2-, 3- and 6-positions, the 2-, 3- and 7-positions, the 2-, 3- and 8-positions, the 2-, 4- and 5-positions, the 2-, 4- and 6-positions, the 2-, 4- and 7-positions, the 2-, 4- and 8-positions, the 2-, 5- and 6-positions, the 2-, 5- and 7-positions, the 2-, 5- and 8-positions, the 2-, 6- and 7-positions, the 2-, 6- and 8-positions, the 3-, 4- and 5-positions, the 3-, 4- and 6-positions, the 3-, 4- and 7-positions, the 3-, 4- and 8-positions, the 3-, 5- and 6-positions, the 3-, 5- and 7-positions, the 3-, 5- and 8-positions, the 3-, 6- and 7-positions, the 3-, 6- and 8-positions, the 3-, 7- and 8-positions, the 4-, 5- and 6-positions, the 4-, 5- and 7-positions, the 4-, 5- and 8-positions, the 4-, 6- and 7-positions, the 4-, 6- and 8-positions, the 4-, 7- and 8-positions, the 5-, 6- and 7-positions, the 5-, 6- and 8-positions, the 5-, 7- and 8-positions, and the 6-, 7- and 8-positions; a trifluoro-1-naphthyl group having three fluorine atoms at the 2-, 3- and 4-positions, the 2-, 3- and 5-positions, the 2-, 3- and 6-positions, the 2-, 3- and 7-positions, the 2-, 3- and 8-positions, the 2-, 4- and 5-positions, the 2-, 4- and 6-positions, the 2-, 4- and 7-positions, the 2-, 4- and 8-positions, the 2-, 5- and 6-positions, the 2-, 5- and 7-positions, the 2-, 5- and 8-positions, the 2-, 6- and 7-positions, the 2-, 6- and 8-positions, the 3-, 4- and 5-positions, the 3-, 4- and 6-positions, the 3-, 4- and 7-positions, the 3-, 4- and 8-positions, the 3-, 5- and 6-positions, the 3-, 5- and 7-positions, the 3-, 5- and 8-positions, the 3-, 6- and 7-positions, the 3-, 6- and 8-positions, the 3-, 7- and 8-positions, the 4-, 5- and 6-positions, the 4-, 5- and 7-positions, the 4-, 5- and 8-positions, the 4-, 6- and 7-positions, the 4-, 6- and 8-positions, the 4-, 7- and 8-positions, the 5-, 6- and 7-positions, the 5-, 6- and 8-positions, the 5-, 7- and 8-positions, and the 6-, 7- and 8-positions; and 2, 3, 4, 5, 6, 7, 8-heptafluoro-1-naphthyl group.

Among the naphthyl groups represented by formula (7), 2-naphthyl groups include, for example: a (trifluoromethyl) -2-naphthyl group having a trifluoromethyl group at the 1-, 3-, 4-, 5-, 6-, 7- or 8-position; a fluoro-2-naphthyl group having a fluorine atom at the 1-, 3-, 4-, 5-, 6-, 7- or 8-position; a bis (trifluoromethyl) -2-naphthyl group having two trifluoromethyl groups at the 1- and 3-positions, the 1- and 4-positions, the 1- and 5-positions, the 1- and 6-positions, the 1- and 7-positions, the 1- and 8-positions, the 3- and 4-positions, the 3- and 5-positions, the 3- and 6-positions, the 3- and 7-positions, the 3- and 8-positions, the 4- and 5-positions, the 4- and 6-positions, the 4- and 7-positions, the 4- and 8-positions, the 5- and 6-positions, the 5- and 7-positions, the 5- and 8-positions, the 6- and 7-positions, the 6- and 8-positions, or the 7- and 8-positions; a difluoro-2-naphthyl group having two fluorine atoms at the 1- and 3-positions, the 1- and 4-positions, the 1- and 5-positions, the 1- and 6-positions, the 1- and 7-positions, the 1- and 8-positions, the 3- and 4-positions, the 3- and 5-positions, the 3- and 6-positions, the 3- and 7-positions, the 3- and 8-positions, the 4- and 5-positions, the 4- and 6-positions, the 4- and 7-positions, the 4- and 8-positions, the 5- and 6-positions, the 5- and 7-positions, the 5- and 8-positions, the 6- and 7-positions, the 6- and 8-positions, or the 7- and 8-positions; a tri(trifluoromethyl)-2-naphthyl group having three trifluoromethyl groups at the 1-, 3- and 4-positions, the 1-, 3- and 5-positions, the 1-, 3- and 6-positions, the 1-, 3- and 7-positions, the 1-, 3- and 8-positions, the 1-, 4- and 5-positions, the 1-, 4- and 6-positions, the 1-, 4- and 7-positions, the 1-, 4- and 8-positions, the 1-, 5- and 6-positions, the 1-, 5- and 7-positions, the 1-, 5- and 8-positions, the 1-, 6- and 7-positions, the 1-, 6- and 8-positions, the 3-, 4- and 5-positions, the 3-, 4- and 6-positions, the 3-, 4- and 7-positions, the 3-, 4- and 8-positions, the 3-, 5- and 6-positions, the 3-, 5- and 7-positions, the 3-, 5- and 8-positions, the 3-, 6- and 7-positions, the 3-, 6- and 8-positions, the 3-, 7- and 8-positions, the 4-, 5- and 6-positions, the 4-, 5- and 7-positions, the 4-, 5- and 8-positions, the 4-, 6- and 7-positions, the 4-, 6- and 8-positions, the 4-, 7- and 8-positions, the 5-, 6- and 7-positions, the 5-, 6- and 8-positions, the 5-, 7- and 8-positions, and the 6-, 7- and 8-positions; a trifluoro-2-naphthyl group having three fluorine atoms at the 1-, 3- and 4-positions, the 1-, 3- and 5-positions, the 1-, 3- and 6-positions, the 1-, 3- and 7-positions, the 1-, 3- and 8-positions, the 1-, 4- and 5-positions, the 1-, 4- and 6-positions, the 1-, 4- and 7-positions, the 1-, 4- and 8-positions, the 1-, 5- and 6-positions, the 1-, 5- and 7-positions, the 1-, 5- and 8-positions, the 1-, 6- and 7-positions, the 1-, 6- and 8-positions, the 3-, 4- and 5-positions, the 3-, 4- and 6-positions, the 3-, 4- and 7-positions, the 3-, 4- and 8-positions, the 3-, 5- and 6-positions, the 3-, 5- and 7-positions, the 3-, 5- and 8-positions, the 3-, 6- and 7-positions, the 3-, 6- and 8-positions, the 3-, 7- and 8-positions, the 4-, 5- and 6-positions, the 4-, 5- and 7-positions, the 4-, 5- and 8-positions, the 4-, 6- and 7-positions, the 4-, 6- and 8-positions, the 4-, 7- and 8-positions, the 5-, 6- and 7-positions, the 5-, 6- and 8-positions, the 5-, 7- and 8-positions, and the 6-, 7- and 8-positions; and 1, 3, 4, 5, 6, 7, 8-heptafluoro-2-naphthyl group.

In the Nile red luminescent compound represented by formula (3), -NR¹R² is an electron-donating group and -Ar is an electron attractive group. Therefore, the hydrogen atom o f -CH- adj acent to Ar- becomes δ +, and the chemical bond between this the carbon atom of -CH- and the carbon atom of the benzene moiety to which the -CH- is bonded becomes a chemical bond like a double bond. As a result, π electron cloud on the skeleton of the Nile red is extended to the substituent, which gives the compound a super resonance effect. Thus, the application of a little energy enables the luminescent compound to emit red light. The novel luminescent compound of this invention is characterized by a structure where R¹-N-R², the electron donating group, provides the π electron cloud with electrons and Ar, the aromatic electron attractive compound, attracts electrons from the π electron cloud. Because this Nile red skeleton has an electronically stable structure and therefore the Nile red luminescent compound is chemically stable, the luminescent compound does not deteriorate even under severe environments, which is a special character of the compound.

A Nile red luminescent compound emitting red light represented by formula (3) may be prepared by the following method.

The compound may be obtained by reacting a Nile red compound represented by formula (2) with the electron attractive aromatic acetonitrile represented by formula (8).

NC-CH₂-Ar (8)

The Nile red compound represented by formula (2) and the electron attractive aromatic acetonitrile react easily by heating them in a solvent. The solvent includes acetic anhydride, acetic acid, an acid anhydride having not more than 5 carbon atoms, an aromatic solvent such as benzene or toluene, a dioxane, etc. The reaction temperature usually ranges between 100°C and 250°C, preferably between 100 and 170°C. After the reaction, purification and separation by an ordinary method will provide the aimed Nile red luminescent compound.

The Nile red luminescent compound in accordance with this invention can easily be produced only by heating a mixture of the Nile red compound and the electron attractive aromatic acetonitrile. A simple production method such as the above-mentioned is an industrial method.

Another Nile red luminescent compound emitting red light in accordance with the present invention is represented by formula (9). 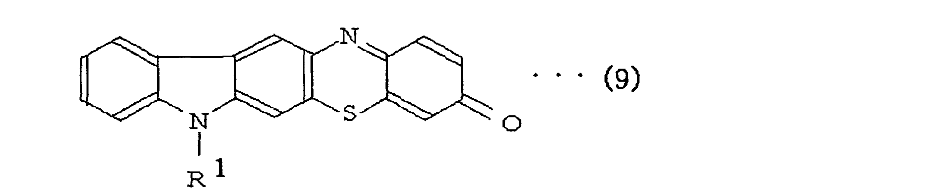 In this formula, R¹ is the same as that defined above.

Still another Nile red luminescent compound emitting red light in accordance with the present invention is represented by formula (10). 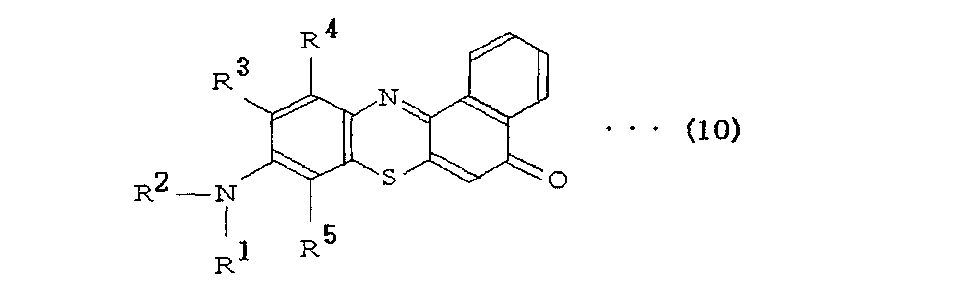 wherein R¹, R², R³, R⁴ and R⁵ are respectively the same as those defined above.

A Nile red luminescent compound of formula (9) may be prepared in the following way.

First, 4-nitrosophenol is reacted with a carbazole, the nitrogen atom of which is bonded with a substituent R¹ (wherein R¹ is the same as that defined above) in accordance with reaction formula (a).

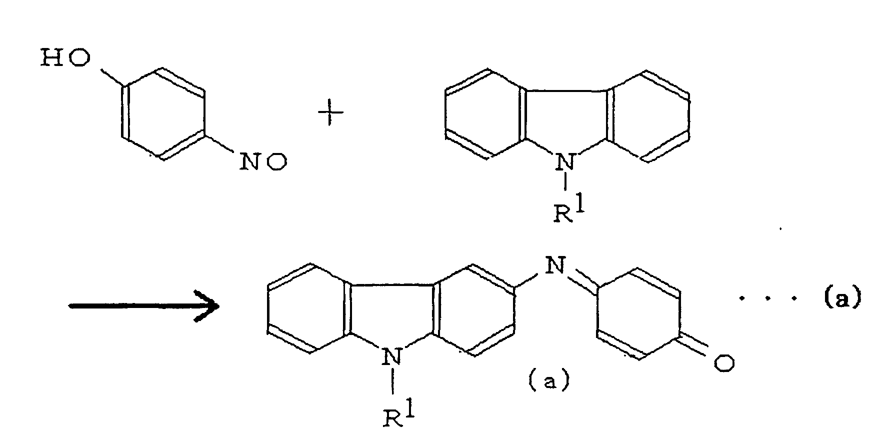

The reaction shown by formula (a) proceeds by stirring the raw materials in a solvent, for example sulfuric acid, at a cooled temperature of -70°C to -40°C, for 1 to 10 hours.

Then, the compound obtained in the previous reaction (a) is reacted with sulfur in a polar aromatic compound solvent, such as o-dichlorobenzene, as shown in reaction formula (b). As a result, the Nile red luminescent compound emitting red light of the present invention, which is represented by formula (9), is prepared. Note that the polar aromatic compound solvent means a solvent of a polar aromatic compound that does not hinder the process of the reaction in accordance with the present invention.

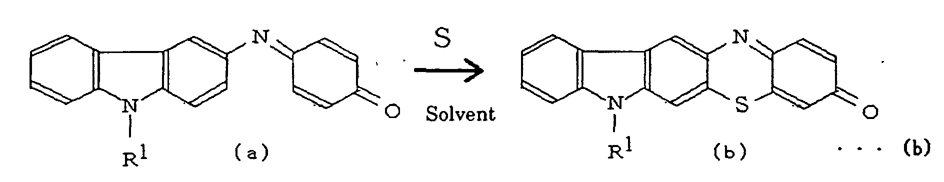

The conditions for the reaction shown in the reaction formula (b) include heating at a temperature of 100°C to 250°C for a half to 5 hours.

On the other hand, the Nile red luminescent compound represented by formula (10) may be prepared in the following way.

First, 1-naphphol is reacted with a 4-nitrosoaniline, the amino group of which is bonded with substituents R¹ and R², wherein each of R¹ and R² is hydrogen atom or an alkyl group, and R¹ and R² may be the same or different from each other, which produces the intermediate compound shown in reaction formula (c).

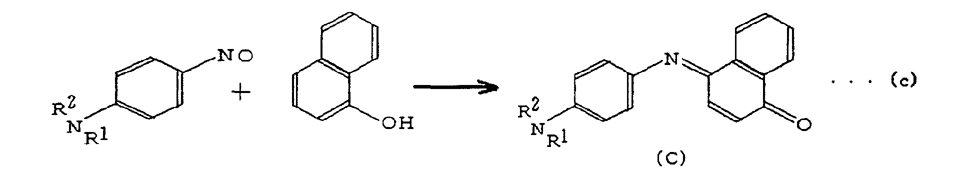

The reaction shown by the reaction formula (c) proceeds by mixing the raw materials in an alkaline hydrophilic solvent, for example an alkaline aqueous solvent while they are being cooled, then stirring them at a temperature of 0 to 100°C for 1 to 10 hours.

Next, the compound obtained in the previous reaction (c) is reacted with sulfur in a polar aromatic compound solvent, such as o-dichlorobenzene, as shown in reaction formula (d), which produces the Nile red luminescent compound emitting red light represented by formula (10) in accordance with the present invention.

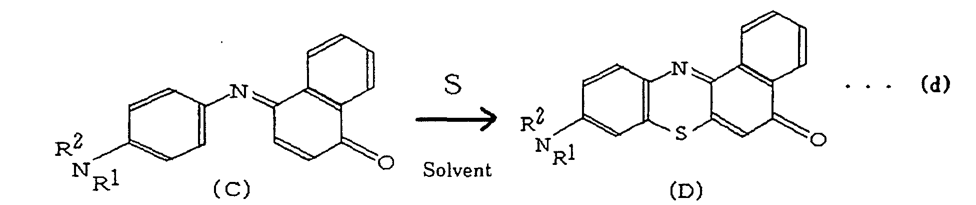

The conditions for the reaction shown by the reaction formula (d) include heating the reactants to 100 to 250°C for 30 minutes to 5 hours.

The Nile red luminescent compound represented by formula (10) is also useful as a raw material or precursor for the Nile red luminescent compound represented by formula (11). 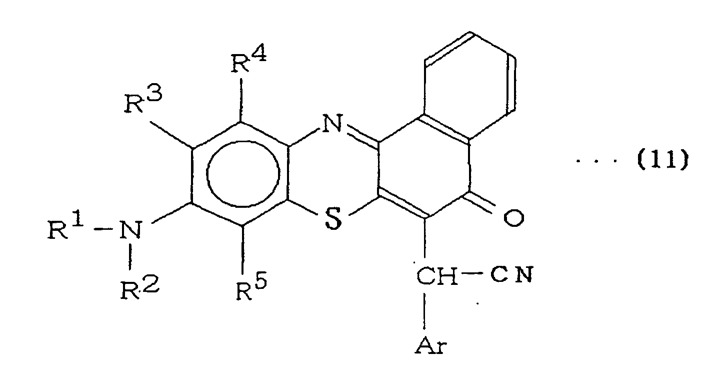 In formula (11), R¹, R², R³, R⁴, R⁵ and Ar mean the same as those defined above.

In the Nile red luminescent compound represented by formula (1), -NR¹R² is an electron-donating group. Since the nitrogen atom and the carbonyl group in the polycyclic skeleton make a quinoid, π electrons are partial to the carbonyl group. Furthermore, Ar, to which at least one fluorine atom is bonded, is a strong electron attractive group, so that the hydrogen atom of -CH- that is bonded with Ar becomes δ⁺. As a result, the single bond between the carbon atom of -CH- and the carbon of the aromatic ring to which -CH- is bonded assumes the characteristic of a double bond, and π electrons on the quinoid is also extended to Ar. This partiality of π electrons enables the luminescent compound to emit red light upon the application of a little energy. In summary, the novel luminescent compound of this invention is characterized by a structure where R¹-N-R², the electron-donating group, provides the π electron cloudwith electrons and Ar, the aromatic electron attractive group, attracts the electrons. Because this Nile red skeleton has an electronically stable structure and therefore the Nile red luminescent compound is chemically stable, the luminescent compound does not deteriorate even under severe environments, which is a special character of the compound.

The Nile red luminescent compound emitting red light represented by formula (11) may be prepared by the following method.

Specifically, the Nile red luminescent compound represented by formula (10) is reacted with the electron attractive aromatic acetonitrile represented by the formula NC-CH₂-Ar, wherein Ar is the same as that defined above.

The Nile red compound represented by formula (10) and the electron attractive aromatic acetonitrile react easily by heating them in a solvent. The solvent includes acetic anhydride, acetic acid, an acid anhydride having not more than 5 carbon atoms, an aromatic solvent such as benzene or toluene, a dioxane, etc. The reaction temperature usually ranges between 100°C and 250°C, preferably between 100 and 170°C. After the reaction, purification and separation by an ordinary method will provide the aimed Nile red luminescent compound.

The Nile red luminescent compound in accordance with this invention can easily be produced only by heating a mixture of the Nile red compound and the electron attractive aromatic acetonitrile. A simple production method such as the above-mentioned is an industrial method.

Also, the Nile red luminescent compound represented by formula (9) can be used as a raw material or precursor for the Nile red luminescent compound having the structure represented by formula (3a). The Nile red luminescent compound emitting red light represented by formula (3a) may be prepared by reacting the Nile red luminescent compound represented by formula (9) with the electron attractive aromatic acetonitrile represented by the formula NC-CH₂-Ar, wherein Ar is the same as that defined above, in the same way as in the preparation of the Nile red luminescent compound (11).

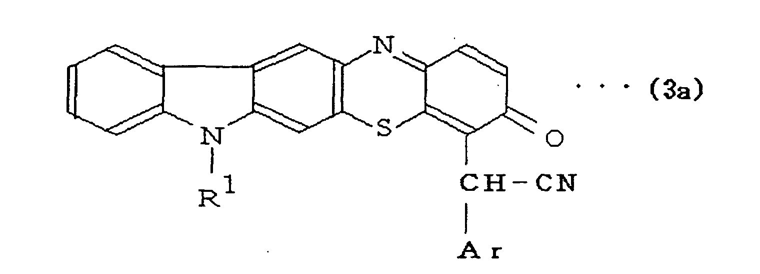

The luminescence element in accordance with the present invention will be explained in the followings.

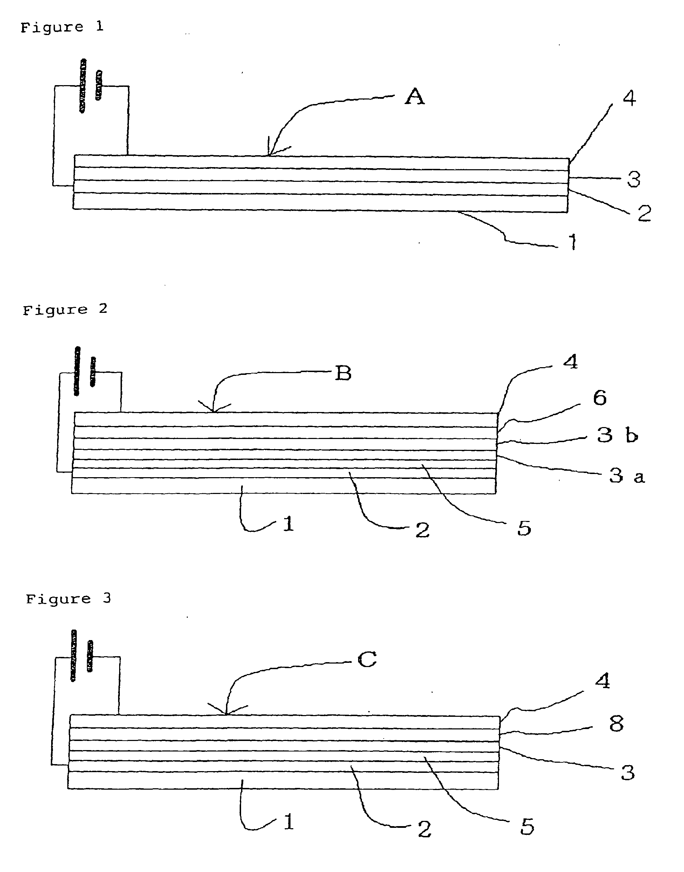

Figure 1 is a schematic illustration that shows the sectional structure of a luminescence element according to the present invention, which is a one-layer type organic EL element. As shown in this figure, the luminescence element A is prepared by layering a light-emitting layer 3 and an electrode layer 4 in this order on a substrate 1 with which a transparent electrode 2 has been provided.

When the luminescence element shown in Figure 1 includes a Nile red luminescent compound emitting red light of the present invention, a blue light-emitting compound and a green light-emitting compound at a balanced composition, it emits white light upon the application of electricity through the transparent electrode 2 and the electrode layer 4. The total amount of the Nile red luminescent compound of the present invention, the blue light-emitting compound and the green light-emitting compound, and the proportion of the amount of the Nile red luminescent compound to that of the blue light-emitting compound to that of the green light-emitting compound, included in the layer 3 to let the element emit white light, vary depending on the kind of each compound. They are decided for each luminescence element depending on the kind of each compound included therein. When the luminescence element is intended to emit red light, the light-emitting layer 3 may include only a Nile red luminescent compound of the present invention. Also, when this luminescence element is intended to emit light of any color other than white and red, the total amount of the compounds and their respective amounts should be changed depending on the color. For example, when the luminescence element including a Nile red luminescent compound of this invention is intended to emit white light, the ratio of the amount of the Nile red luminescent compound to that of the blue light-emitting compound to that of the green light-emitting compound is usually 5-200:10-100:50-20000 in weight, preferably 10-100:50-500: 100-10000.

The blue light-emitting compound includes a diphenylvinyl biphenol compound emitting blue light and a stilbene compound emitting blue light. An example of preferable diphenylvinyl biphenol compounds emitting blue light is DPVBi represented by formula (16).

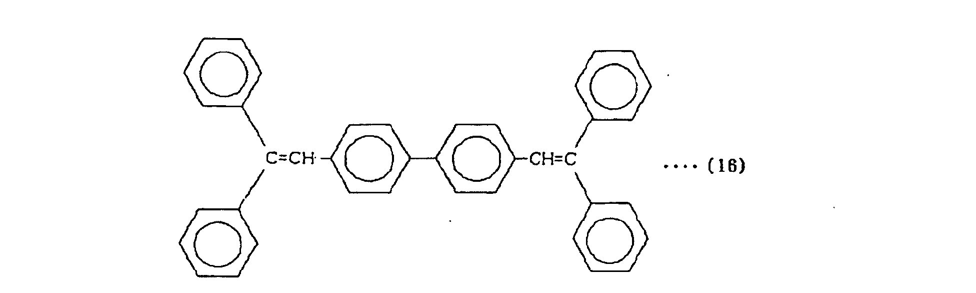

For the green light-emitting compound is suitable a coumarin compound emitting green light, an indophenol compound emitting green light and an indigo compound emitting green light. The coumarin compound represented by formula (17) is preferable among them.

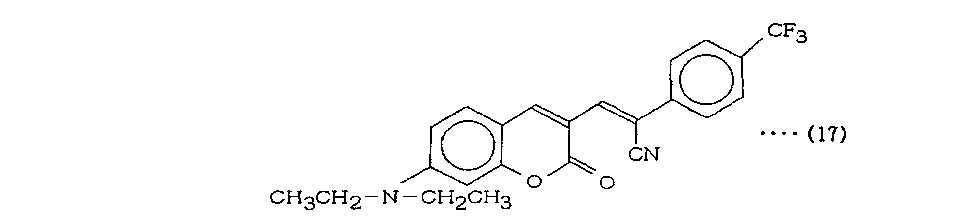

Upon the application of an electric field between the transparent electrode 2 and the electrode layer 4, electrons are injected from the electrode layer 4 and positive holes are injected from the transparent electrode 2. In the light-emitting layer 3, the electrons are recombined with positive holes, which causes the energy level to return to the valence band from the conduction band. This transition of the energy level is accompanied by emission of the energy differential as light.

The luminescence element A shown in Figure 1, when it is shaped to a planar one with a large area, may be used as a planar illuminator, for example a large-area wall illuminator emitting white light when f ixed on a wall, or a large-area ceiling illuminator emitting white light when fixed on a ceiling. This light-emitting element may be used as a planar light source in place of a point light source, such as a conventional bulb, and a line light source, such as a conventional fluorescent lamp. In particular, this white light-emitting illuminator can suitably be used to light up walls, ceilings and floors in dwelling rooms, offices and passenger trains, or to make them emit light. Moreover, this luminescence element A may be suitable for the backlight used in displays of computers, cellular phones and ATMs. Furthermore, this illuminator may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. It may also be used as a light source for brake lights of vehicles such as cars. In addition, because the light-emitting element A includes a Nile red luminescent compound of the present invention, which has the special chemical structure, in the light-emitting layer, it has a long life. Therefore, light sources employing the luminescence element A will naturally have a long life.

As understood from the foregoing, when the light-emitting layer of the luminescence element A includes a Nile red luminescent compound emitting red light of the present invention and not the blue light-emitting compound or the green light-emitting compound, the luminescence element A emits clear red light.

The luminescence element A may also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order. Because this luminescence element A does not include mercury, it is an ecological light source and may be a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate of which surface is insulated, for example, through the formation of an insulating layer thereon.

When the substrate 1 is opaque, the luminescence element, which includes the blue light-emitting compound, the green light-emitting compound and a Nile red luminescent compound of the present invention, is a single-faced illuminator that emits white light from one side of the element. On the other hand, when the substrate 1 is transparent, the luminescence element is a double-faced illuminator that emits white light from both of the substrate and the surface layer opposite to the substrate.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 includes a Nile red luminescent compound of the present invention when the layer 3 is intended to emit red light. It includes the blue light-emitting compound and the green light-emitting compound in addition to a Nile red luminescent compound of the present invention when it is intended to emit white light. The light-emitting layer 3 may be a high polymer film prepared by dissolving the Nile red luminescent compound emitting red light of the present invention, or the blue light-emitting compound, the green light-emitting compound and the Nile red luminescent compound of the present invention in a high polymer. Also, the light-emitting layer 3 may be a deposited film which is prepared by depositing the Nile red luminescent compound of the present invention, or the blue light-emitting compound, the green light-emitting compound and the Nile red luminescent compound of the present invention on the transparent electrode 2.

Examples of the high polymer for the high polymer film are a polyvinyl carbazole, a poly (3-alkylthiophen), a polyimide including an arylamide, a polyfluorene, a polyphenylene vinylene, poly-α-methylstyrene, and a copolymer of vinylcarbazole and α-methylstyrene. Of them, a polyvinyl carbazole is preferable.

The amount of the Nile red luminescent compound emitting red light, or the blue light-emitting compound, the green light-emitting compound and the Nile red luminescent compound in the high polymer film is, typically, 0.01 to 2 weight%, preferably, 0.05 to 0.5 weight%.

The thickness of the high polymer film ranges, typically, between 30 nm and 500 nm, preferably between 100 nm and 300 nm. When the thickness is too small, the amount of the emitted light may be insufficient. On the other hand, when the thickness is too large, voltage required to drive the element may be too high, which is not desirable. Besides, the large thickness may reduce the flexibility of the element necessary to shape a planar, tubular, curved or ring article.

A typical example of forming the high polymer film on the transparent electrode may be the application of a solution of the Nile red luminescent compound or the mixture of the blue light-emitting compound, the green light-emitting compound and the Nile red luminescent compounddissolved in a suitable solvent onto the transparent electrode. The application method includes, for example, a spin cast method, a brush-coating method, etc.

When the light-emitting layer 3 is made of a deposited film, the thickness of the film is typically 0.1 to 100 nm, although it varies depending on the structure of the light-emitting layer 3. When the thickness is too small or too large, the deposited film layer will have the same problems as the high polymer film layer.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode may easily be formed on the surface of light-emitting layer 3, which, in turn, has been formed on substrate 1, by the technique of metal deposition.

When either of the deposition or the application is employed for the formation of the light-emitting layer, a buffer layer should be inserted between the electrode layer and the light-emitting layer.

Materials for the buffer layer are, for example, an alkaline metal compound such as lithium fluoride, an alkaline earth metal compound such as magnesium fluoride, an oxide such as an aluminum oxide, and 4,4'-biscarbazole biphenyl (Cz-TPD). Also, materials for forming a buffer layer between the cathode made of ITO, etc. and the organic layer are, for example, m-MTDATA (4,4',4"-tris(3-methylphenyl-phenylamino)triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the luminescence element, improve the quantum efficiency of luminescence, and achieve an increase in the luminance of the emitted light.

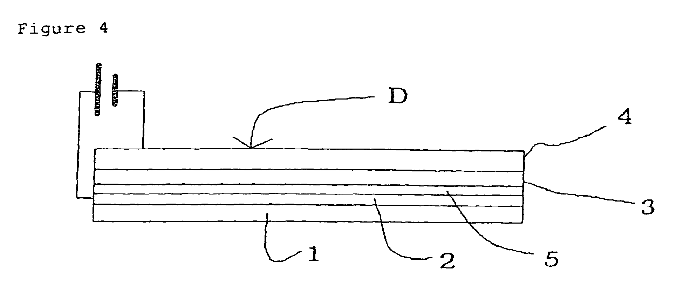

Next, the second example of the luminescence element in accordance with this invention is shown in Figure 2. This figure is an illustration showing the sectional layer structure of an example of the luminescence element, which is a multi-layer organic EL element.

As shown in Figure 2, the luminescence element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescence element A in Figure 1.

The light-emitting layer of the luminescence element B comprises the light-emitting sublayers 3a and 3b. The light-emitting sublayer 3a is a deposited film including light-emitting compounds. The light-emitting sublayer 3b is a DPVBi layer that functions as a host.

Examples of the hole-transporting substance included in the hole-transporting layer 5 are a triphenylamine compound such as N,N'-diphenyl-N,N'-di(m-tolyl)-benzidine (TPD) and α-NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenyl)-1,3,4-oxadiazole and 2,5-bis(1-naphthyl)-1,3,4-oxadiazole, and 2, 5-bis (5'-tert-butyl-2'-benzoxazolyl) thiophene. Also, a metal complex material such as quinolinol aluminum complex (Alq3), benzoquinolinol beryllium complex (Bebq2) may be used suitably.

The luminescence element B shown in Figure 2 employs Alq3 as electron-transporting substance in the electron-transporting layer 6.

The thickness of each layer is the same as that in a known multi-layer organic EL element.

The luminescence element B in Figure 2 functions and emits light in the same ways as the luminescence element A in Figure 1. Therefore, the luminescence element B has the same uses as the luminescence element A.

The third example of the luminescence element of this invention is shown in Figure 3. This figure is an illustration showing the sectional layer structure of an example of the luminescence element, which is a multi-layer organic EL element.

The luminescence element C shown in Figure 3 comprises a substrate 1, and a transparent electrode 2, a hole-transporting layer 5, a light-emitting layer 3, an electron-transporting layer 8, and an electrode layer 4, wherein the transparent electrode and the layers are laid on substrate 1 one by one in this order.

The luminescence element C functions in the same way as the luminescence element B.

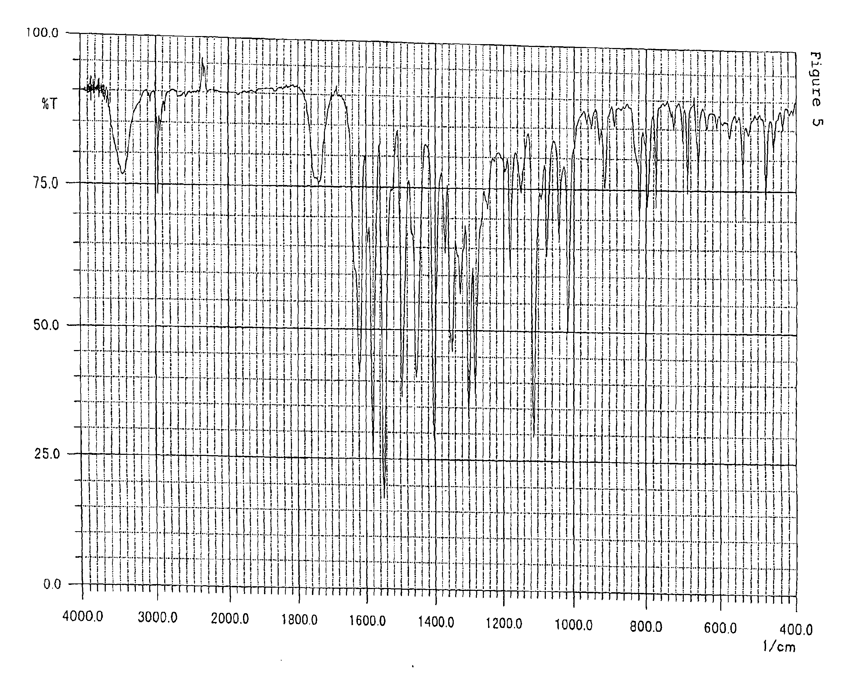

Another example of the luminescence element of this invention is shown in Figure 4. The luminescence element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescence elements, other than those shown in Figures 1-4, is a two-layer low molecular weight organic luminescence element having a hole-transporting layer that includes a hole-transporting substance and an electron-transporting light-emitting layer that includes a Nile red luminescent compound of the invention laid on the hole-transporting layer, these layers being sandwiched between a cathode, which is the transparent electrode formed on the substrate, and an anode, which is the electrode layer. A specific example of this embodiment is a two-layer pigment-inj ected luminescence element comprising a hole-transporting layer and a light-emitting layer that includes a host pigment and a Nile red luminescent compound of this invention as a guest pigment, wherein the light-emitting layer is laid on the hole-transporting layer and these layers are sandwiched between the cathode and the anode. Another example is a two-layer organic luminescence element comprising a hole-transporting layer that includes a hole-transporting substance and an electron-transporting light-emitting layer that is prepared through a co-deposition of a red light-emitting compound of the invention and an electron-transporting substance, the latter layer being laid on the former, and these two layers being sandwiched between the cathode and the anode. A specific example of the second embodiment is a two-layer pigment-injected luminescence element comprising a hole-transporting layer and an electron-transporting light-emitting layer that includes a host pigment and a Nile red luminescent compound of this invention as a guest pigment, wherein the light-emitting layer is laid on the hole-transporting layer and these layers are sandwiched between the cathode and the anode. A further example is a three-layer organic luminescence element comprising a hole-transporting layer, a light-emitting layer including a Nile red luminescent compound emitting red light of this invention that is laid on the hole-transporting layer, and an electron-transporting layer that is laid on the light-emitting layer, these layers being sandwiched between the cathode and the anode.

The electron-transporting layer typically comprises 50-80% by weight of a polyvinyl carbazole (PVK), 5-40% by weight of an electron-transporting luminescent agent, and 0.01-20% by weight of a Nile red luminescent compound of the present invention. The composition within these ranges results in the emission of red light at a strong luminance.

Also, it is preferred if the light-emitting layer includes, as a sensitizing agent, rubrene, especially both of rubrene and Alq3.

A red light-emitting element utilizing a Nile red luminescent compound emitting red light of the present invention, or a white light-emitting element utilizing the blue light-emitting compound, the green light-emitting compound and a Nile red luminescent compound of the present invention may generally be used for an organic EL element driven by direct current, and also by pulses and alternating current.

### Examples

### (Working Example 1)

### <Synthesis of a Nile Red Luminescent Compound>

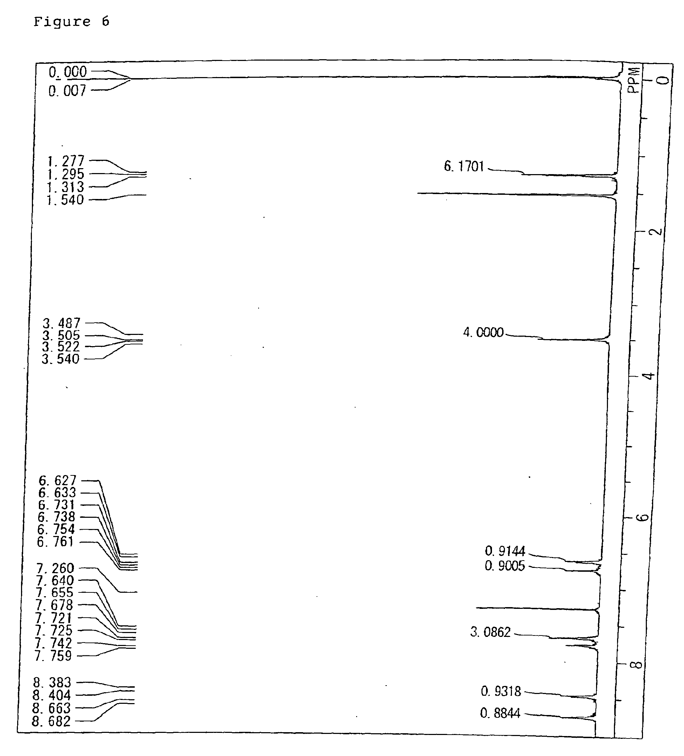

5.0g (15.7 mmol) of Nile red, 5.63g (23.6 mmol) of diethyl bromomalonate, and 250 ml of acetic anhydride were placed in a 500 ml pear-shaped flask. The solution in the pear-shaped flask was heated in a silicone oil bath to 135°C and allowed to react for 2.5 hours. Acetic anhydride was distilled away with an evaporator and solids were obtained. A column, which had been filled with silica gel, was charged with the solids, and the solid matter was purified with benzene as a developer. 200 mg of a deep green solid matter was obtained. The melting point of the solid matter was 204-205°C. An IR spectrum of this deep green solid matter is shown in Figure 5, and an NMR chart of the solid matter is shown in Figure 6. The results of elemental analysis of this product are as follows.
Calculated values: C: 60.47, H: 4.31, N: 7.05, O: 8.05, Br: 20.11
Found values: C: 59.19, H: 4.24, N: 6.43, O: 8.36, Br: 21.61
Based on these results, the deep green solid matter was identified as a Nile red luminescent compound emitting red light that has the structure represented by formula (18).

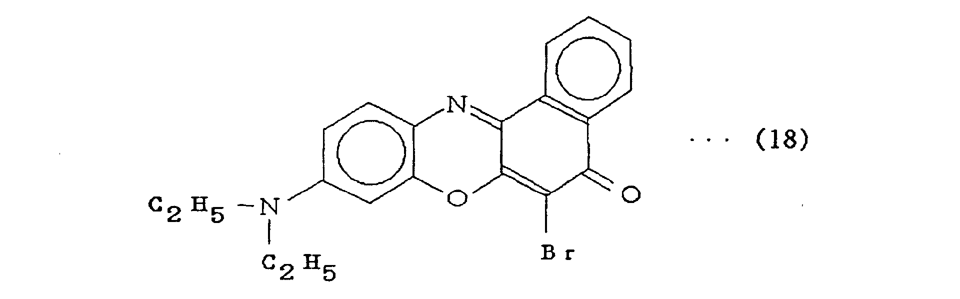

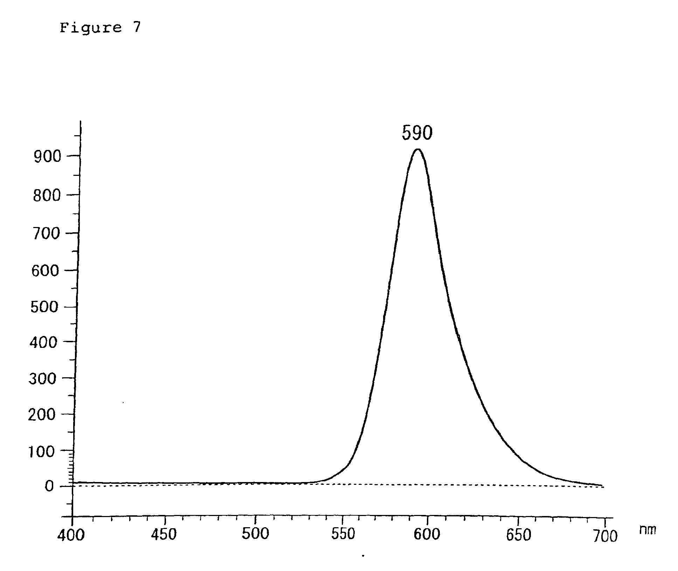

A sample solution was prepared by dissolving the deep green solid matter represented by formula (18) in benzene, so that the concentration of the compound was 10 mg/L. This sample solution was loaded in a model F-4500 spectrofluorophotometer, a product by Shimadzu Corporation, and the fluorescence spectrum of the solution was measured under the following conditions. The measured spectrum is shown in Figure 7.

### Conditions of Measurement

Measuring mode: Wavelength scanning
Exciting wavelength: 365 nm
Wavelength at which the emission of fluorescence started: 400 nm
Wavelength at which the emission of fluorescence ended: 700 nm
Scanning speed: 1200 nm/min.
Slit on the side of excitation: 5.0 nm
Slit on the side of fluorescence emission: 5.0 nm
Photomal voltage: 700 V

As understood from Figure 7, the fluorescence of the Nile red luminescent compound obtained in this example covers a light range of which wavelengths are from 550 nm to 700 nm.

### <Luminescence element Having a Light-emitting Layer Applied by Spin-coating>

In a 5ml graduated flask were placed 70 mg of polyvinyl carbazole, 30 mg of 2,5-bis(1-naphthyl)-1,3,4-oxadiazole (BND), and 0.15 mg of the deep green solid matter, represented by formula (18), obtained in the previous experiment. Dichloroethane was added to the mixture so that the total volume of the mixture was 5 ml. Thus, a solution containing the Nile red luminescent compound was prepared. This solution was sufficiently homogenized by irradiating it with ultrasound for 20 minutes using a model US-2 ultrasonic cleaner, a product by SND Co. An ITO substrate, a product by Sanyo Shinku Industries, Co., Ltd., the dimensions of which were 50 x 50 mm and the transparent electrode of which had a thickness of 200 µm, was ultrasonically cleaned in acetone for 10 minutes and then in 2-propanol for 10 minutes. The substrate was blow-dried with nitrogen. Then, the substrate was cleaned by ultraviolet-light irradiation for 5 minutes at a wavelength of 254 nm with a photo surface processor. The solution containing the Nile red luminescent compound was dropped onto the obtained ITO substrate and a film was formed on the surface of the substrate by spin-coating at 1,500 rpm for 3 seconds with a model 1H-D7 spin coater, a product by Mikasa Co., Ltd, so that the thickness of the dried film would be 100 µm. The substrate with the film was dried for 30 minutes in a constant temperature bath of 50°C. The electrode of an aluminum alloy, a product by Kojundo Chemical Laboratory, Co., Ltd., in which the weight ratio of Al to Li was 99:1, was deposited on the film under 4 x 10⁻⁶ Torr with a vacuum metallizer (Model VDS-M2-46, produced by DIAVAC Limited). The thickness of the electrode was about 150 nm. Thus an EL element as shown in Figure 1 was obtained.

The luminance and the chromaticity of the EL element were measured with a Fast BM-7 measuring apparatus produced by TOPCON Corporation, with the voltage being raised gradually. When the voltage was 20 V and the current was 10. 65 mA, the luminance was 1095. 00 Cd/m², the value on the x-axis in CIE chromaticity, which will be called "chromaticity X" hereinafter, was 0.6642, and the value on the y-axis in E chromaticity, which will be called "chromaticity Y", was 0.3270.

### <Luminescence element Having a Deposited Light-emitting Layer>

An ITO substrate, a product by Sanyo Shinku Industries, Co., Ltd., the dimensions of which were 50 x 50 mm, was ultrasonically cleaned in acetone for 10 minutes and then in 2-propanol for 10 minutes. The substrate was blow-dried with nitrogen. Then, the substrate was cleaned by ultraviolet-light irradiation for 5 minutes at a wavelength of 254 nm with a photo surface processor produced by SEN LIGHTS CORPORATION.

The cleaned ITO substrate was loaded on a vacuum metallizer (Model VDS-M2-46, produced by DIAVAC Limited). Then, 45 nm of the TPD layer, and 40 nm of a layer comprising Alq3 doped with 2% of the Nile red luminescent compound represented by formula (18) were deposited on the substrate under 4 x 10⁻⁶ Torr in this order. The light-emitting layer was formed in this way. Finally, the electrode of an aluminum alloy, a product by Kojundo Chemical Laboratory, Co., Ltd., in which the weight ratio of Al to Li was 99:1, was deposited on the light-emitting layer. The thickness of the electrode was about 150 nm. Thus a red light-emitting EL element as shown in Figure 1 was obtained.

The luminance and the chromaticity of the EL element were measured with a Fast BM-7 measuring apparatus produced by TOPCON Corporation, with the voltage being raised gradually. When the voltage was 16 V and the current was 33. 81 mA, the luminance was 84.38 Cd/m², chromaticity X 0.6717, and chromaticity Y 0.3283.

### (Working Example 2)

### <Synthesis of another Nile Red Luminescent Compound>

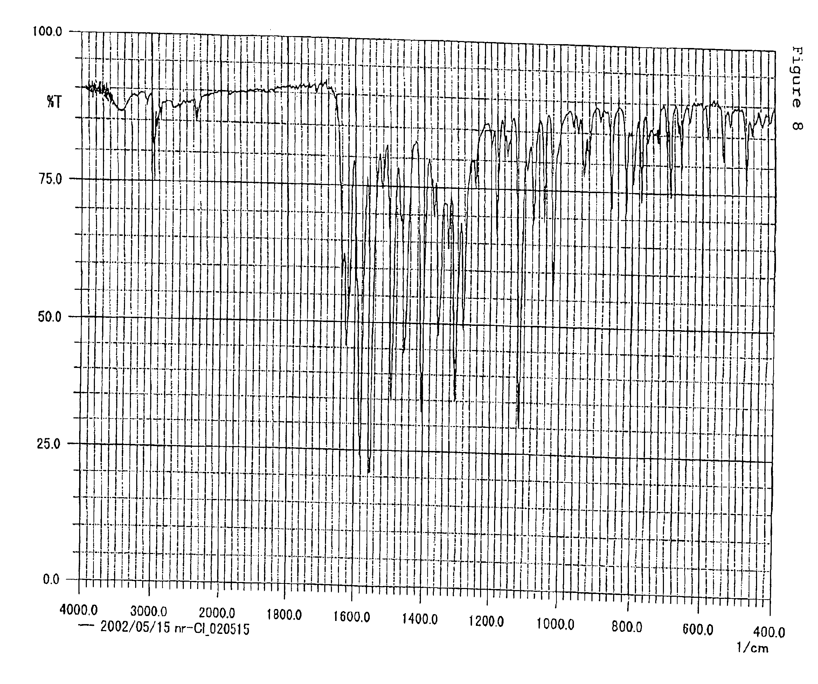

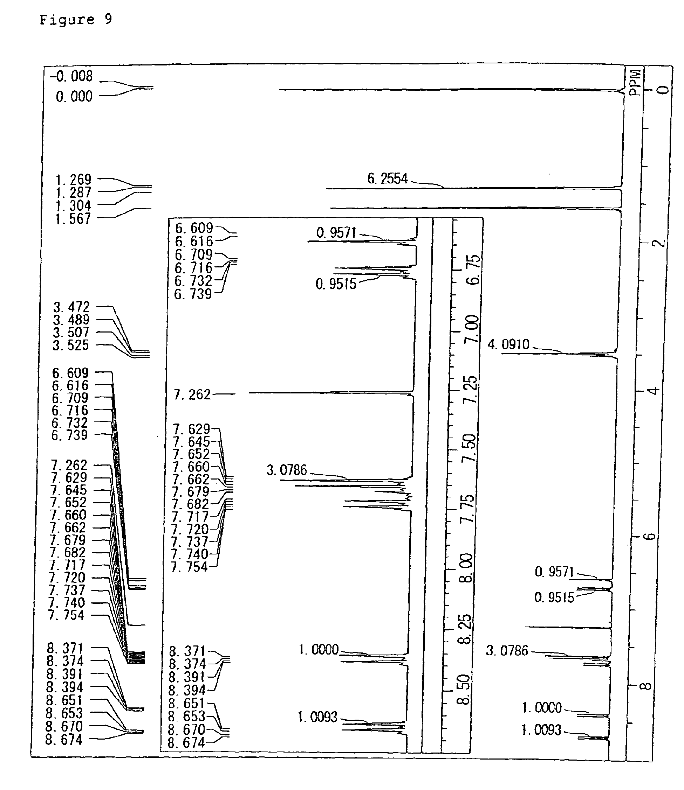

1.27g (4.0 mmol) of Nile red, 0.53g (4.0 mmol) of N-chlorosuccinimide, and 200 ml of carbon tetrachloride were placed in a 500 ml pear-shaped flask. The solution in the pear-shaped flask was heated in a silicone oil bath to 110°C and allowed to react for 2 hours. Carbon tetrachloride was distilled away with an evaporator and the remaining solids were dissolved in 700 ml of chloroform. This chloroform solution was washed with ion-exchanged water, and then dried with sodium sulfate. The dried solution was concentrated with an evaporator. The obtained solid was purified by a column chromatography that used silica gel for the filler and benzene for the developer. 830 mg of a black solid matter was obtained. The melting point of the solid matter was 200-222°C. An IR spectrum of this product is shown in Figure 8 and an NMR chart thereof is shown in Figure 9. The results of elemental analysis of this product are as follows.
Calculated values: C: 68.08, H: 4.86, N: 7.94, O: 9.07, Cl: 10.05
Found values: C: 67.79, H: 5.01, N: 7.89, O: 8.89, Cl: 10.31

Based on these results, the obtained product was identified as a Nile red luminescent compound emitting red light that has the structure represented by formula (19).

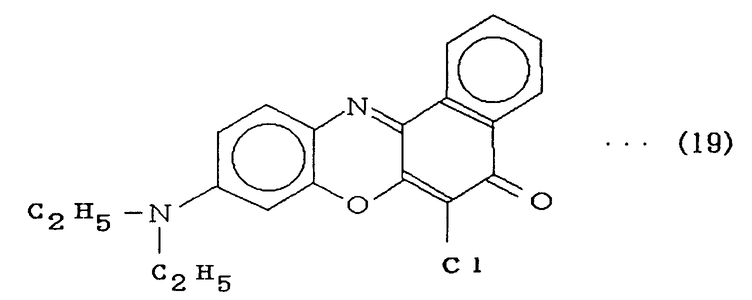

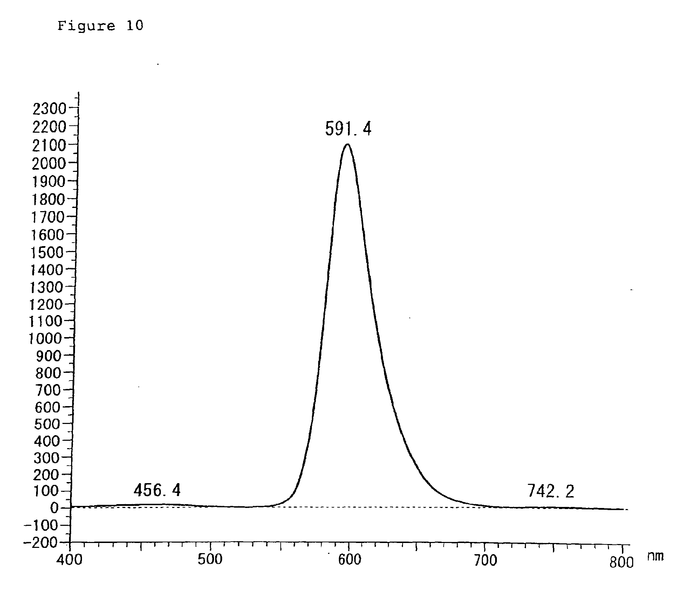

A sample solution was prepared by dissolving the solid matter represented by formula (19) in benzene, so that the concentration of the compound was 10 mg/L. The fluorescence spectrum of the sample solution was measured with the same method as in Working Example 1 under the following conditions. The measured spectrum is shown in Figure 10.

### Conditions of Measurement

Measuring mode: Wavelength scanning
Exciting wavelength: 365 nm
Wavelength at which the emission of fluorescence started: 400 nm
Wavelength at which the emission of fluorescence ended: 800 nm
Scanning speed: 1200 nm/min.
Slit on the side of excitation: 5.0 nm
Slit on the side of fluorescence emission: 5.0 nm
Photomal voltage: 700 V

As understood from Figure 10, the fluorescence of the Nile red luminescent compound obtained in this example covers a light range of which wavelengths are from 550 nm to 700 nm.

### <Luminescence element Having a Light-emitting Layer Applied by Spin-coating>

In a 5ml graduated flask were placed 70 mg of polyvinyl carbazole, 30mg of 2,5-bis (1-naphthyl)-1,3,4-oxadiazole (BND), and 0.3 mg of the black solid matter, represented by formula (19), obtained in the previous experiment. Dichloroethane was added to the mixture so that the total volume of the mixture was 5 ml. Thus, a solution containing the Nile red luminescent compound was prepared. This solution was sufficiently homogenized by irradiating it with ultrasound for 20 minutes using a model US-2 ultrasonic cleaner, a product by SND Co. An ITO substrate, a product by Sanyo Shinku Industries, Co., Ltd., the dimensions of which were 50 x 50 mm and the transparent electrode of which had a thickness of 200 µm, was ultrasonically cleaned in acetone for 10 minutes and then in 2-propanol for 10 minutes. The substrate was blow-dried with nitrogen. Then, the substrate was cleaned by ultraviolet-light irradiation for 5 minutes at a wavelength of 254 nm with a photo surface processor. The solution containing the Nile red luminescent compound was dropped onto the obtained ITO substrate and a film was formed on the surface of the substrate by spin-coating at 1,500 rpm for 3 seconds with a model 1H-D7 spin coater, a product by Mikasa Co., Ltd, so that the thickness of the dried film would be 100 µm. The substrate with the film was dried for 30 minutes in a constant temperature bath of 50°C. The electrode of an aluminum alloy, a product by Kojundo Chemical Laboratory, Co., Ltd., in which the weight ratio of Al to Li was 99:1, was deposited on the film under 4 x 10⁻⁶ Torr with a vacuum metallizer (Model VDS-M2-46, produced by DIAVAC Limited). The thickness of the electrode was about 150 nm. Thus an EL element as shown in Figure 1 was obtained.

The luminance and the chromaticity of the EL element were measured with a Fast BM-7 measuring apparatus produced by TOPCON Corporation, with the voltage being raised gradually. When the voltage was 20 V and the current was 10.06 mA, the luminance was 1215.00 Cd/m², chromaticity X 0.6229, and chromaticity Y 0.3599.

### <Luminescence element Having a Deposited Light-emitting Layer>

An ITO substrate, a product by Sanyo Shinku Industries, Co., Ltd., the dimensions of which were 50 x 50 mm, was ultrasonically cleaned in acetone for 10 minutes and then in 2-propanol for 10 minutes. The substrate was blow-dried with nitrogen. Then, the substrate was cleaned by ultraviolet-light irradiation for 5 minutes at a wavelength of 254 nm with a photo surface processor produced by SEN LIGHTS CORPORATION.

The cleaned ITO substrate was loaded on a vacuum metallizer (Model VDS-M2-46, produced by DIAVAC Limited). Then, 45 nm of the TPD layer, and 40 nm of a layer comprising Alq3 doped with 2% of the Nile red luminescent compound represented by formula (19) were deposited on the substrate under 4 x 10⁻⁶ Torr in this order. The light-emitting layer was formed in this way. Finally, the electrode of an aluminum alloy, a product by Kojundo Chemical Laboratory, Co., Ltd., in which the weight ratio of Al to Li was 99:1, was deposited on the light-emitting layer. The thickness of the electrode was about 150 nm. Thus a red light-emitting EL element as shown in Figure 1 was obtained.

The luminance and the chromaticity of the EL element were measured with a Fast BM-7 measuring apparatus produced by TOPCON Corporation, with the voltage being raised gradually. When the voltage was 13 V and the current was 38.53 mA, the luminance was 361 Cd/m², chromaticity X 0. 6489, and chromaticity Y 0.3511.

### (Working Example 3)

### <Synthesis of Nile Red Luminescent Compound A>

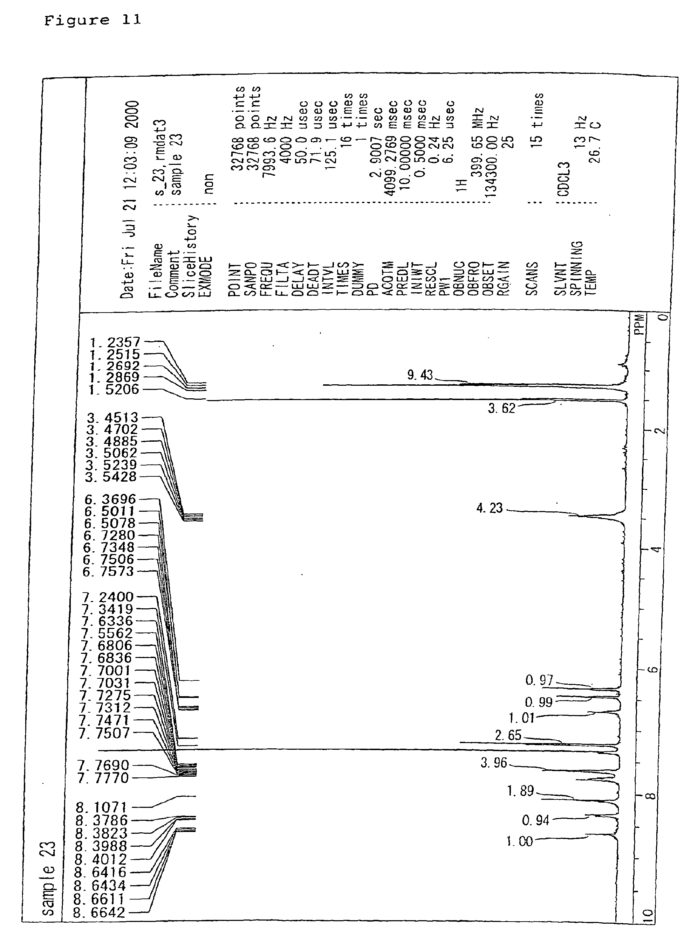

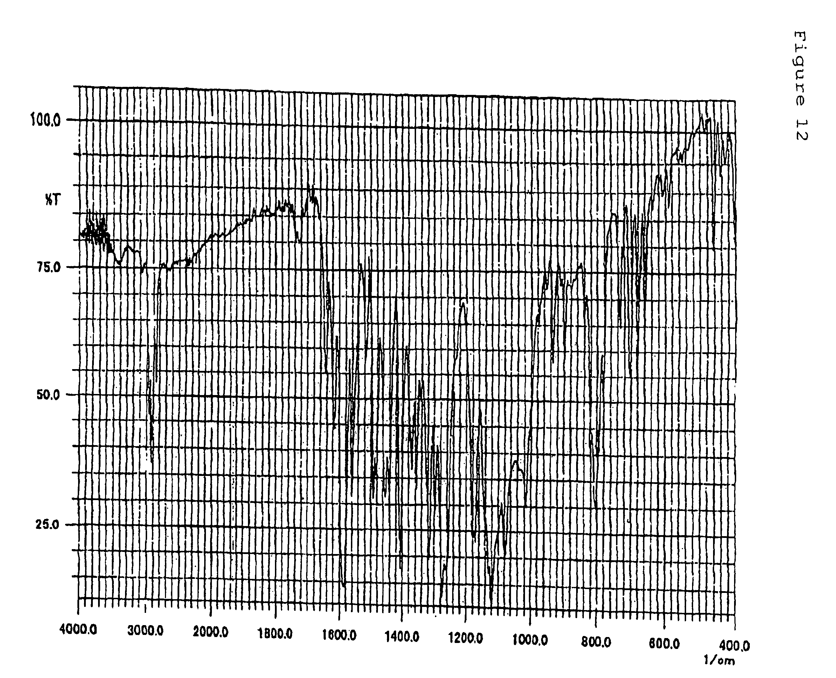

0.50g (1.57 mmol) of Nile red, 0.40g (1.57 mmol) of 3,5-bis(trifluoromethyl)phenylacetonitrile, and 25 ml of acetic anhydride were placed in a 100 ml pear-shaped flask. The solution in the pear-shaped flask was heated in a silicone oil bath to 135° C and allowed to react for 4 hours. Acetic anhydride was distilled away with an evaporator and the remaining was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel for the filler and benzene for the developer. 30 mg of violaceous solid was obtained in a 12% yield. The melting point of the product was 257-260°C. A ¹H-NMR spectrum and an IR spectrum of this product are shown in Figures 11 and 12. Based on these results, the obtained product was identified as the chemical compound represented by formula (20).

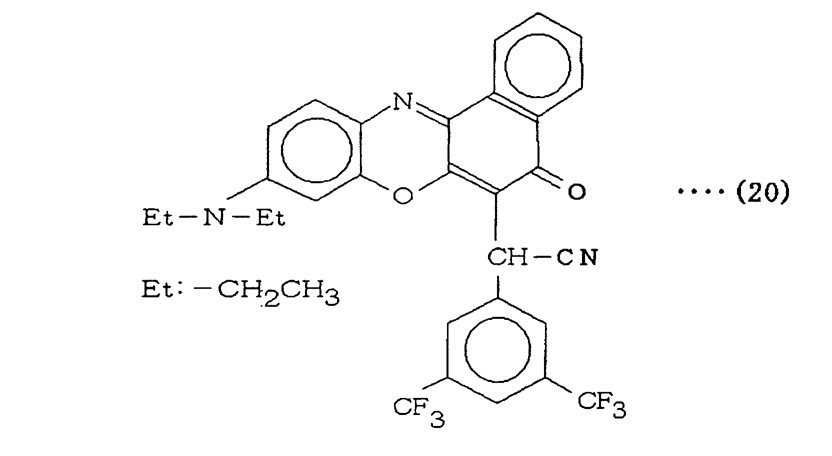

### (Working Example 4)

### <Synthesis of Nile Red Luminescent Compound B>

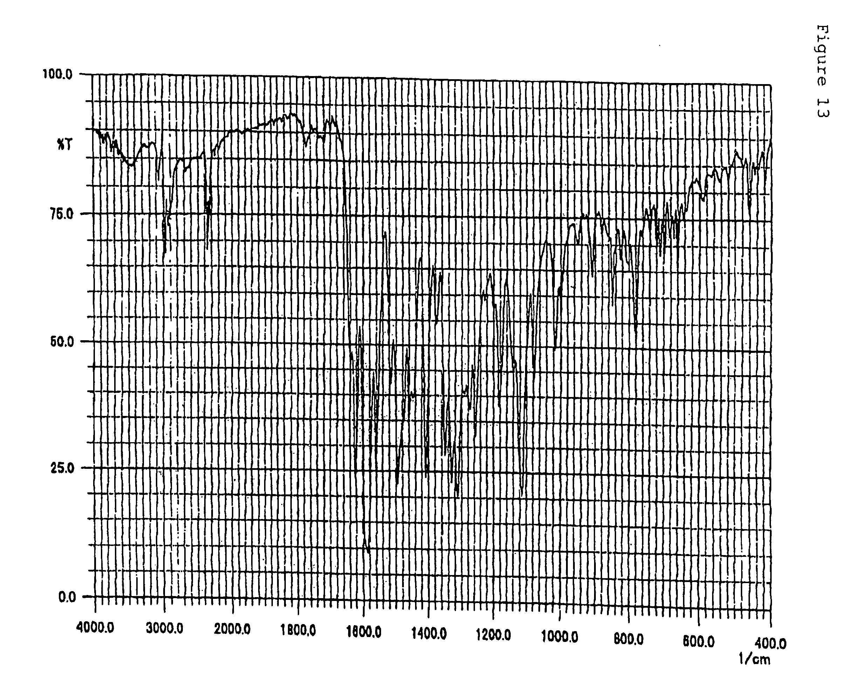

0.50g (1.57 mmol) of Nile red, 0.35g (1.57 mmol) of 2,3-difluoro-4-(trifluoromethyl)phenylacetonitrile, and 25 ml of acetic anhydride were placed in a 100 ml pear-shaped flask. The solution in the pear-shaped flask was heated in a silicone oil bath to 135°C and allowed to react for 3 hours. Acetic anhydride was distilled away with an evaporator and the remaining was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel for the filler and benzene for the developer. 10 mg of violaceous solid was obtained in a 6.4% yield. The melting point of the product was 172-174°C. An IR spectrum of this product is shown in Figure 13. Based on these results, the obtained product was identified as the chemical compound represented by formula (21).

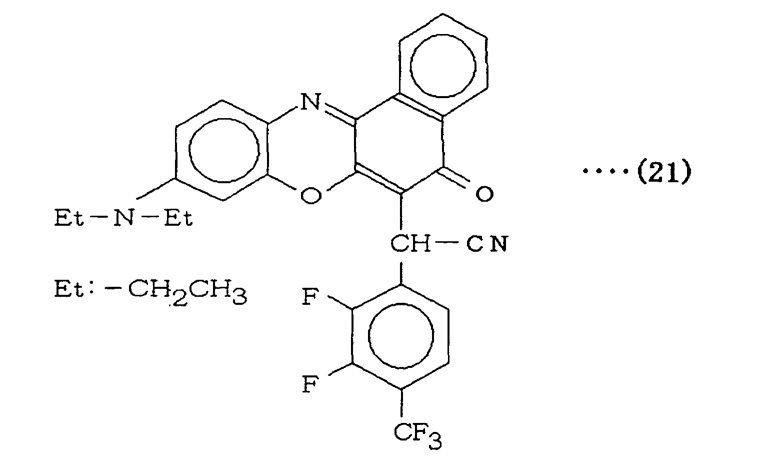

### (Working Example 5)

In a 5 ml graduated flask were placed 70 mg of polyvinyl carbazole, a product produced by Kanto Kagaku Co., Ltd., which polyvinyl carbazole will be abbreviated to PVK, 29 mg of 2,5-bis(1-naphthyl)-1,3,4-oxadiazole synthesized by the inventors, which will be abbreviated to BND, and 1 mg of Nile red luminescent compound A. Dichloroethane was added to the mixture so that the total volume of the mixture was 5 ml. Thus, a solution containing the Nile red luminescent compound was prepared. This solution was sufficiently homogenized by irradiating it with ultrasound for 20 minutes using a model US-2 ultrasonic cleaner, a product by SND Co. An ITO substrate, a product by Sanyo Shinku Industries, Co., Ltd., of which dimensions were 50 x 50 mm, was ultrasonically cleaned in acetone for 10 minutes and then in 2-propanol for 10 minutes. The substrate was blow-dried with nitrogen. Then, the substrate was cleaned by ultraviolet-light irradiation for 30 seconds at a wavelength of 172 nm with a UV irradiator produced by M. D. Excimer Inc. The solution containing the Nile red luminescent compound was dropped onto the obtained ITO substrate and a film was formed on the surface of the substrate by spin-coating at 1, 500 rpm for 3 seconds with a model 1H-D7 spin coater, a product by Mikasa Co., Ltd. The substrate with the film was dried for 30 minutes in a constant temperature bath of 50°C. The electrode of an aluminum alloy, a product by Kojundo Chemical Laboratory, Co., Ltd., in which the weight ratio of Al to Li was 99:1, was deposited on the film under 4 x 10⁻⁶ Torr with a vacuum metallizer (Model VDS-M2-46, produced by DIAVAC Limited). The thickness of the electrode was 1, 500 Å. Thus an EL element was obtained.

The luminance and the chromaticity of the EL element were measured with a Fast BM-7 measuring apparatus produced by TOPCON Corporation, with the voltage being raised gradually. The results are shown in Table 2.

### (Working Example 6)

In a 5 ml graduated flask were placed 68 mg of PVK, 31.2 mg of 2-(4-tert-butylphenyl-5-(4-biphenylyl)-1,3,4-oxadiazole, which will be abbreviated to PBD, and 0.8 mg of Nile red luminescent compound A. Dichloroethane was added to the mixture so that the total volume of the mixture was 5 ml. An EL element was prepared from the obtained solution containing the Nile red luminescent compound, and the luminance and the chromaticity thereof were measured with the same methods as in Working Example 5 above. The results are shown in Table 2.

### (Working Example 7)

In a 5 ml graduated flask were placed 63.7 mg of PVK, 35.5 mg of 2,5-bis(5'-tert-butyl-2'-benzoxazolyl)thiophene, which will be abbreviated to BBOT, and 0.8 mg of Nile red luminescent compound A. Dichloroethane was added to the mixture so that the total volume of the mixture was 5 ml. An EL element was prepared from the obtained solution containing the Nile red luminescent compound, and the luminance and the chromaticity thereof were measured with the same methods as in Working Example 5 above. The results are shown in Table 2.

### (Working Example 8)

In a 5 ml graduated flask were placed 64.0 mg of PVK, 35.6 mg of BBOT, and 0.4 mg of Nile red luminescent compound A. Dichloroethane was added to the mixture so that the total volume of the mixture was 5 ml. An EL element was prepared from the obtained solution containing the Nile red luminescent compound, and the luminance and the chromaticity thereof were measured with the same methods as in Working Example 5 above. The results are shown in Table 2.

### (Comparative Example 1)

In a 5 ml graduated flask were placed 68.2 mg of PVK, 31.3 mg of PBD, and 0.5 mg of Nile red. Dichloroethane was added to the mixture so that the total volume of the mixture was 5 ml. An EL element was prepared from the obtained solution containing Nile red, and the luminance and the chromaticity thereof were measured with the same methods as in Working Example 5 above. The results are shown in Table 2.

**Table 2**

| Amount mg | | W.Ex.5 | W.Ex.6 | W.Ex.7 | W.Ex.8 | C.Ex.1 |
|---|---|---|---|---|---|---|
| PVK | | 70.0 | 68.0 | 63.7 | 64.0 | 68.2 |
| BND | | 29.0 | | | | |
| PBD | | | 31.2 | | | 31.3 |
| BBOT | | | | 35.5 | 35.6 | |
| Pigment A | | 1.0 | 0.8 | 0.8 | 0.4 | |
| Nile red compound | | | | | | 0.5 |
| The highest luminance cd/m² | | 2454.4 | 2215.0 | 2629.2 | 3475.6 | 2137.0 |
| Chromaticity | X | 0.6417 | 0.6307 | 0.6330 | 0.6278 | 0.5402 |
| | Y | 0.3477 | 0.3535 | 0.3610 | 0.3648 | 0.4324 |

### (Working Example 9)

In a 5 ml graduated flask were placed 70.1 mg of polyvinyl carbazole, a product produced by Kanto Kagaku Co., Ltd., which polyvinyl carbazole will be abbreviated to PVK, 29.3 mg of 2,5-bis(1-naphthyl)-1,3,4-oxadiazole synthesized by the inventors, which will be abbreviated to BND, and 0.61 mg of Nile red luminescent compound A. Dichloroethane was added to the mixture so that the total volume of the mixture was 5 ml. Thus, a solution containing the Nile red luminescent compound was prepared. This solution was sufficiently homogenized by irradiating it with ultrasound for 20 minutes using a model US-2 ultrasonic cleaner, a product by SND Co. An ITO substrate, a product by Sanyo Shinku Industries, Co., Ltd., of which dimensions were 50 x 50 mm, was ultrasonically cleaned in acetone for 10 minutes and then in 2-propanol for 10 minutes. The substrate was blow-dried with nitrogen. Then, the substrate was cleaned by ultraviolet-light irradiation for 30 seconds at a wavelength of 172 nm with a UV irradiator produced by M. D. Excimer Inc. The solution containing the Nile red luminescent compound was dropped onto the obtained ITO substrate and a film was formed on the surface of the substrate by spin-coating at 1,500 rpm for 3 seconds with a model 1H-D7 spin coater, a product by Mikasa Co., Ltd. The substrate with the film was dried for 30 minutes in a constant temperature bath of 50°C. The electrode of an aluminum alloy, a product by Kojundo Chemical Laboratory, Co., Ltd., in which the weight ratio of Al to Li was 99:1, was deposited on the film under 4 x 10⁻⁶ torr with a vacuum metallizer (Model VDS-M2-46, produced by DIAVAC Limited). The thickness of the electrode was 1,500 Å. Thus an EL element was obtained.

The luminance and the chromaticity of the EL element were measured with a Fast BM-7 measuring apparatus produced by TOPCON Corporation, with the voltage being raised gradually. The results are shown in Table 3.

### (Working Example 10)

In a 5 ml graduated flask were placed 69.9 mg of PVK, 29.1 mg of BND, 0.4 mg of rubrene and 0.6 mg of Nile red luminescent compound A. Dichloroethane was added to the mixture so that the total volume of the mixture was 5 ml. An EL element was prepared from the obtained solution containing the Nile red luminescent compound, and the luminance and the chromaticity thereof were measured with the same methods as in Example 9 above. The results are shown in Table 3.

### (Working Example 11)

The steps of Working Example 9 were repeated, except that Nile red luminescent compound B was used instead of Nile red luminescent compound A. The results of the measurements are shown in Table 3.

### (Working Example 12)

### <Synthesis of Nile Red Luminescent Compound C>

The steps of Working Example 3 were repeated, except that 1.57 mmol of 2,4-bis(trifluoromethyl)phenylacetonitrile was used instead of 3,5-bis(trifluoromethyl)phenylacetonitrile. Thus, Nile red luminescent compound C represented by formula (22) was synthesized.

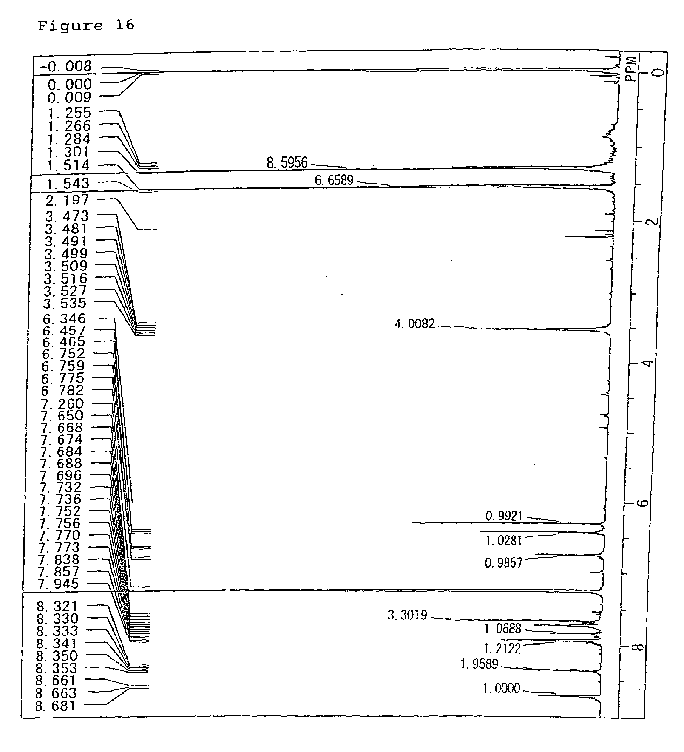

A ¹H-NMR spectrum of this product is shown in Figure 16.

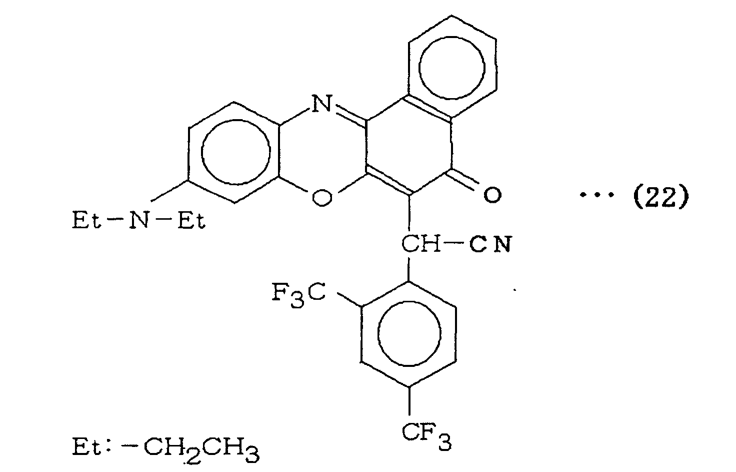

The steps of Working Example 9 were repeated, except that Nile red luminescent compound C was used instead of Nile red luminescent compound A. Thus, an EL element was obtained. The luminance and the chromaticity of this EL element were measured with the same methods as in Working Example 9. The results of the measurements are shown in Table 3.

**Table 3**

| Amount mg | | W.Ex.9 | W.Ex.10 | W.Ex.11 | W.Ex.12 |
|---|---|---|---|---|---|
| PVK | | 70.1 | 69.9 | 70.1 | 70.1 |
| BND | | 29.3 | 29.1 | 29.3 | 29.3 |
| Rubrene | | | 0.4 | | |
| Luminescent compound A | | 0.6 | 0.6 | | |
| Luminescent compound B | | | | 0.6 | |
| Luminescent compound C | | | | | 0.6 |
| The highest luminance cd/m2 | | 3006.0 | 2015.0 | 1349.0 | 2985.0 |
| Chromaticity | X | 0.6248 | 0.6694 | 0.6294 | 0.6218 |
| | Y | 0.3453 | 0.3152 | 0.3458 | 0.3636 |

### (Working Example 13)

An ITO substrate, which had been cleaned in the same way as in Working Example 9, was set in a vacuum metallizer, and N,N'-diphenyl-N,N-di(m-tolyl)-benzidine (TPD) was deposited on the substrate under not more than 1 x 10⁻⁶ torr. The thickness of the deposited film was 60 nm. Then, a mixture of tris (8-quinolinate) aluminum (Alq3) and Nile red luminescent compound A, wherein the amount of the latter was 1.7 weight%, was further deposited on the surface of the TPD film, so that the thickness of the film of the mixture was 31 nm. Finally, aluminum electrode was deposited in a thickness of 150 nm. Thus, an EL element was prepared.

The highest luminance and the chromaticity of this EL element were measured with the same method as in Working Example 9. The results are shown in Table 4.

### (Working Example 14)

An EL element was prepared in the same way as in Working Example 13, except that the amount of Nile red luminescent compound A in the mixture of Alq3 and compound A was 1.6 weight%, and the mixture was deposited on the substrate so that the thickness of the film was 40 nm. The results are shown in Table 4.

**Table 4**

| | | W.Ex.13 | W.Ex.14 |
|---|---|---|---|
| Thickness of film (nm) | TPD | 60 | 60 |
| | Alq3 + Luminescent Cpd. A | 31 | 40 |
| Concentrations in the mixture | Alq3 | 98.5 | 98.4 |
| | Luminescent compound A | 1.5 | 1.6 |
| The highest luminance (cd/m²) | | 4623 | 11990 |
| Chromaticity | X | 0.6536 | 0.6497 |
| | Y | 0.3184 | 0.3276 |

### (Working Example 15)

In a 5 ml graduated flask were placed 70.0 mg of poly(N-vinyl carbazole), a product produced by Kanto Kagaku Co., Ltd., which poly (N-vinyl carbazole) will be abbreviated to PVK, 14.85 mg of 2,5-bis(1-naphthyl)-1,3,4-oxadiazole, a product produced by Lancaster, which will be abbreviated to BND, 0.05 mg of Nile red luminescent compound A, 0.10 mg of a green light-emitting pigment represented by formula (17), which will be called "pigment B", and 15.0 mg of a blue light-emitting pigment represented by formula (16), which will be abbreviated to DPVBi. Dichloroethane was added to the mixture so that the total volume of the mixture was 5 ml. This solution was sufficiently homogenized by irradiating it with ultrasound for 20 minutes using a model US-2 ultrasonic cleaner, a product by SND Co.

An ITO substrate was ultrasonically cleaned in acetone for 10 minutes and then in IPA for 10 minutes. The substrate was blow-dried with nitrogen. Then, the substrate was cleaned by ultraviolet-light irradiation for 5 minutes with a model PL16-110 photo surface processor produced by SEN LIGHTS CORPORATION. The solution was dropped onto the obtained ITO substrate and a film was formed on the surface of the substrate by spin-coating with a model 1H-D7 spin coater, a product by Mikasa Co., Ltd. The substrate with the film was dried for 30 minutes in a constant temperature bath of 50°C. The electrode of an aluminum alloy, a product by Kojundo Chemical Laboratory, Co., Ltd., in which the weight ratio of Al to Li was 99:1, was deposited on the film under a pressure of not more than 10⁻⁶ Torr with a vacuum metallizer (Model VDS-M2-46, produced by DIAVAC Limited). The thickness of the electrode was about 150 nm. Thus an EL element was obtained.

The optical properties of the EL element were measured with a Fast BM-7 measuring apparatus produced by TOPCON Corporation. The results are shown in Table 5. As understood from Table 5, the combination of the Nile red luminescent compound in accordance with this invention, the green light-emitting pigment and the blue light-emitting pigment could provide an EL element capable of emitting white light.

### (Working Example 16)

In a 5 ml graduated flask were placed 70.1 mg of PVK, 14.85 mg of BND, 0.04 mg of Nile red luminescent compound A, 0.10 mg of the green light-emitting pigment, which was the same as that used in Working Example 15, and 15.0 mg of the blue light-emitting pigment, which was the same as that used in Working Example 15. Dichloroethane was added to the mixture so that the total volume of the mixture was 5 ml. An EL element was prepared in the same way as in Working Example 15 and the optical properties thereof were measured. The results are shown in Table 5. As understood from Table 5, the combination of the Nile red luminescent compound in accordance with this invention, the green light-emitting pigment and the blue light-emitting pigment could provide an EL element capable of emitting white light.

### (Working Example 17)

In a 5 ml graduated flask were placed 70.0 mg of PVK, 20.0 mg of BND, 0.02 mg of Nile red luminescent compound A, 0. 03 mg of the green light-emitting pigment, which was the same as that used in Working Example 15, and 9.95 mg of the blue light-emitting pigment, which was the same as that used in Working Example 15. Dichloroethane was added to the mixture so that the total volume of the mixture was 5 ml. An EL element was prepared in the same way as in Working Example 15 and the optical properties thereof were measured. The results are shown in Table 5. As understood from Table 5, the combination of the Nile red luminescent compound in accordance with this invention, the green light-emitting pigment and the blue light-emitting pigment could provide an EL element capable of emitting white light.

**Table 5**

| | W. Ex. 15 | W. Ex. 16 | W. Ex. 17 |
|---|---|---|---|
| PVK | 70.0 | 70.1 | 70.0 |
| BND | 14.85 | 14.85 | 20.0 |
| Pigment A | 0.05 | 0.04 | 0.02 |
| Pigment B | 0.10 | 0.10 | 0.03 |
| DPVBi | 15.0 | 15.0 | 9.95 |
| The highest luminance (cd/m²) | 4867.0 | 3631.0 | 2819.0 |
| Chromaticity X | 0.3788 | 0.3616 | 0.3101 |
| Y | 0.3547 | 0.3399 | 0.3035 |

### (Working Example 18)

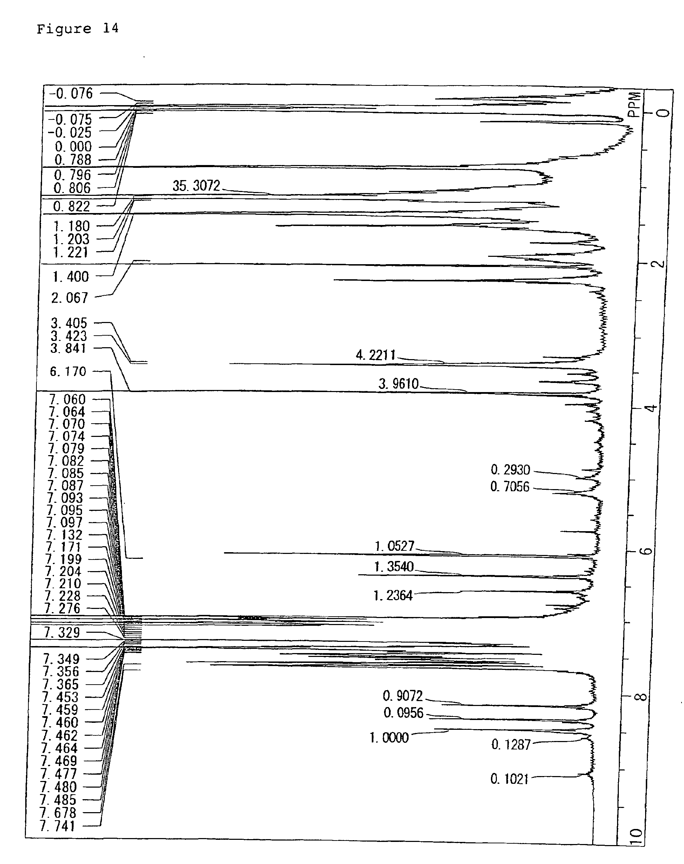

0.92g (2.89 mmol) of Nile red, 1.0g (4.33 mmol) of fluorinated phenylacetonitrile (1) represented by formula (23), and 50 ml of acetic anhydride were placed in a 100 ml pear-shaped flask. The solution in the pear-shaped flask was heated in a silicone oil bath to 135°C and allowed to react for 1 hour. Acetic anhydride was distilled away with an evaporator and the residue was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel for the filler and benzene for the developer. 40 mg of violaceous solids were obtained. The melting point of the solids was 235-240°C. An NMR chart of this product is shown in Figure 14. Based on these results, the obtained product was identified as Nile red luminescent compound D represented by formula (24).

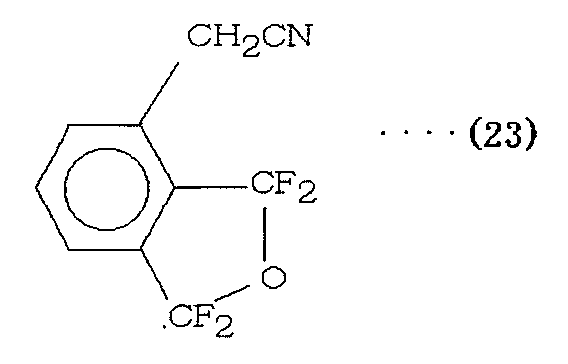

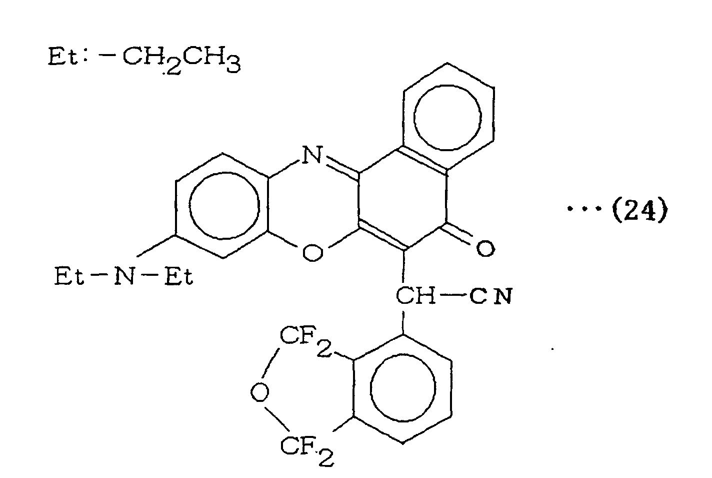

### (Working Example 19)

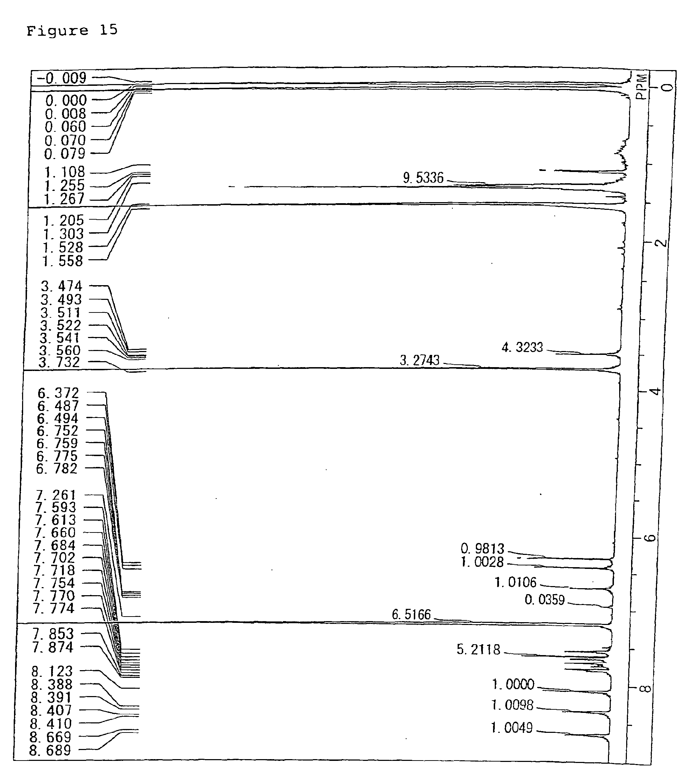

0.92g (2.89 mmol) of Nile red, 1.0g (4.33 mmol) of fluorinated phenylacetonitrile (2) represented by formula (25), and 50 ml of acetic anhydride were placed in a 100 ml pear-shaped flask. The solution in the pear-shaped flask was heated in a silicone oil bath to 135°C and allowed to react for 1 hour. Acetic anhydride was distilled away with an evaporator and the residue was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel for the filler and benzene for the developer. 50 mg of violaceous solids were obtained. The melting point of the product was 250-252°C. An NMR chart of this product is shown in Figure 15. Based on these results, the obtained product was identified as Nile red luminescent compound E represented by formula (26).

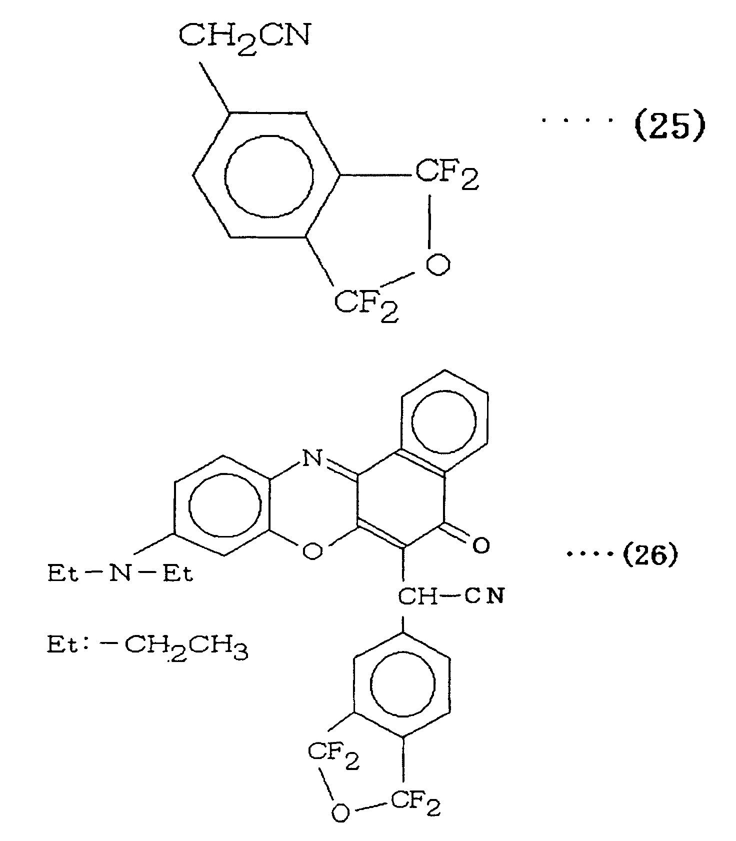

### <Organic EL Element Utilizing Nile Red Luminescent Compound D>

In a 5 ml graduated flask were placed 70. 0 mg of polyvinyl carbazole, which will be abbreviated to PVK hereinafter, 29.7 mg of BND, and 0.3 mg of the Nile red luminescent compound represented by formula (24). Dichloroethane was added to the mixture so that the total volume of the mixture was 5 ml. Then, the solution including the Nile red luminescent compound was prepared. An EL element was made from this solution in the same manner as in Working Example 9.

The highest luminance and the chromaticity of the EL element were measured with a Fast BM-7 measuring apparatus produced by TOPCON Corporation, with the voltage being raised gradually. When the voltage was 17 V and the current was 9.07 mA, the luminance was 1575 Cd/m² and the value on the x-axis in CIE chromaticity was 0. 6552. When the voltage was 18 V and the current was 11.84 mA, the luminance was 1815 Cd/m² and the value on the x-axis in CIE chromaticity was 0.6563. When the voltage was 19 V and the current was 13.98 mA, the luminance was 1702 Cd/m² and the value on the x-axis in CIE chromaticity was 0. 6559. When the voltage was 20 V and the current was 16.84 mA, the luminance was 1505 Cd/m² and the value on the x-axis in CIE chromaticity was 0.6517.

### <Organic EL Element Utilizing Nile Red Luminescent Compound E>

An ITO substrate was cleaned in the same manner as in Working Example 9 and the cleaned ITO substrate was set in a vacuum metallizer. Under a reduced pressure of not more than 1 x 10⁻⁶ torr, N,N'-diphenyl-N,N-di(m-tolyl)-benzidine (TPD) was vapor-deposited on the substrate, which results in a film having a thickness of 60 nm. Then, a mixture of tris (8-quinolinate) aluminum (Alq3) and Nile red luminescent compound E represented by formula (26), wherein the amount of the latter was 2 weight%, was deposited on the surface of the TPD-applied substrate, so that the thickness of the upper film was 31 nm. Finally, the aluminum electrode of 150 nm in thickness was formed on the surface of the upper film by deposition. Thus an EL element was prepared.

The luminance and the chromaticity of the EL element were measured in the same manner as in Working Example 9. When the voltage was 27 V and the current was 15.37 mA, the luminance was 3660 Cd/m², and the value on the x-axis in CIE chromaticity was 0.6266.

### (Working Example 20)

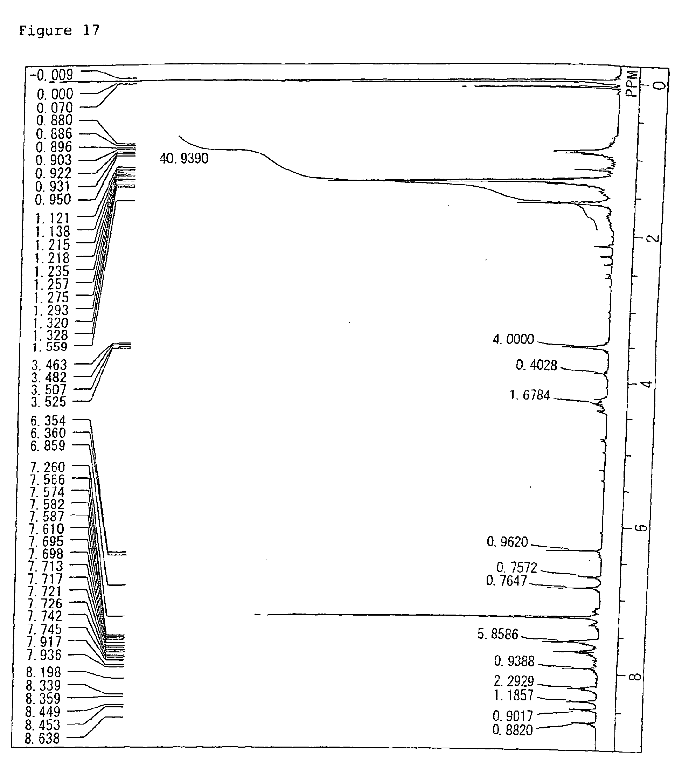

0.92g (2.89 mmol) of Nile red, 1.0g (4.33 mmol) of fluorinated phenylacetonitrile (3) represented by formula (27), and 60 ml of acetic anhydride were placed in a 100 ml pear-shaped flask. The solution in the pear-shaped flask was heated in a silicone oil bath to 135°C and allowed to react for 3.5 hours. Acetic anhydride was distilled away with an evaporator and the residue was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel and benzene. 10 mg of violaceous solid was obtained. An NMR chart of this product is shown in Figure 17. Based on these results, the obtained product was identified as Nile red luminescent compound F represented by formula (28).

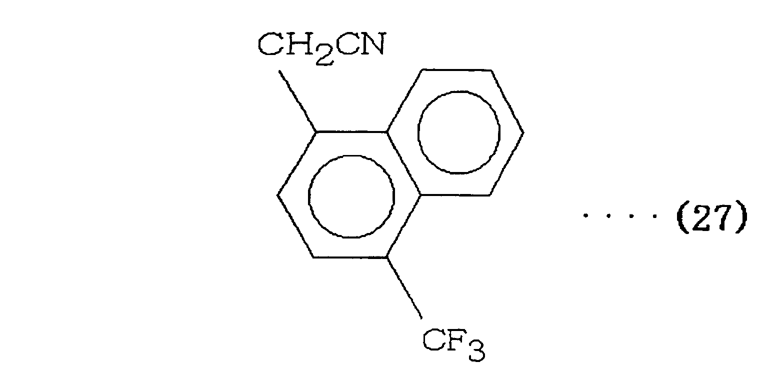

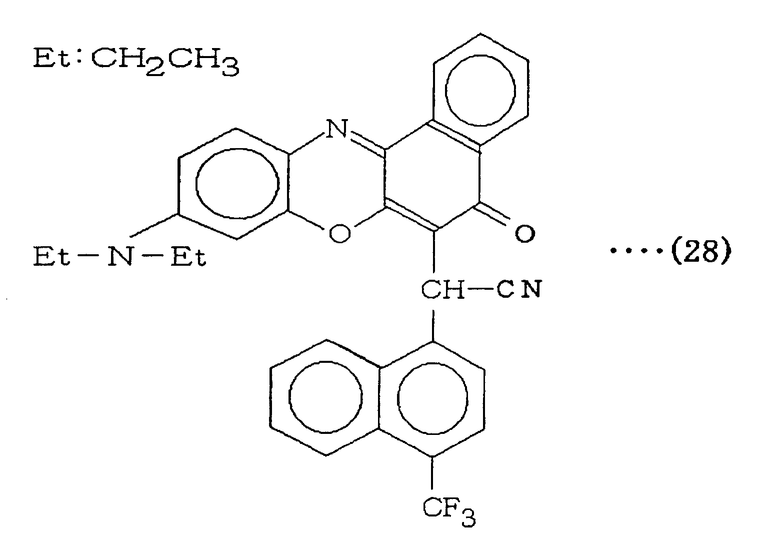

### (Working Example 21)

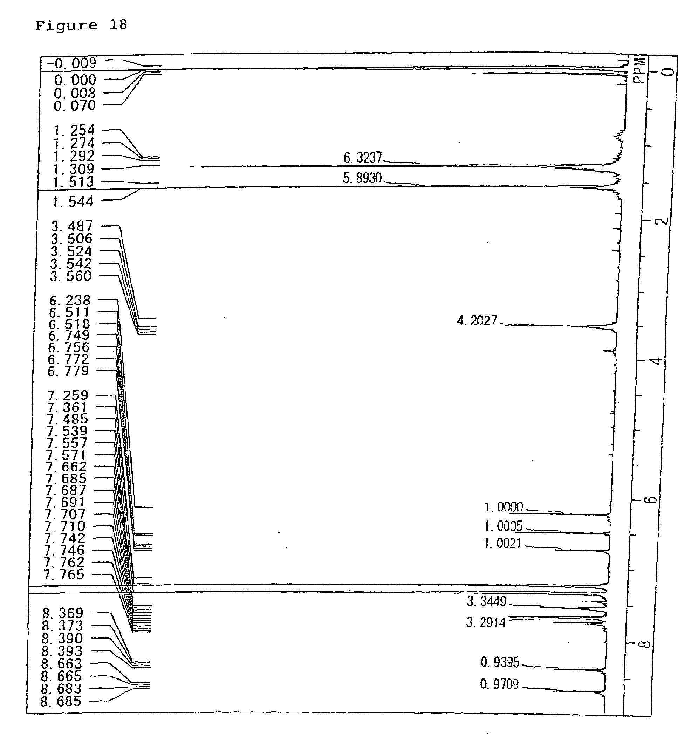

1.04g (3.28 mmol) of Nile red, 1.0g (4.92 mmol) of 3-fluoro-4- (trifluoromethyl)phenylacetonitrile, and 50 ml of acetic anhydride were placed in a 100 ml pear-shaped flask. The solution in the pear-shaped flask was heated in a silicone oil bath to 135°C and allowed to react for 2.5 hours. Acetic anhydride was distilled away with an evaporator and the residue was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel and benzene. 40 mg of violaceous solids were obtained. The melting point of the violaceous solids was 215-220°C. An NMR chart of this product is shown in Figure 18. Based on these results, the obtained product was identified as Nile red luminescent compound G represented by formula (29).

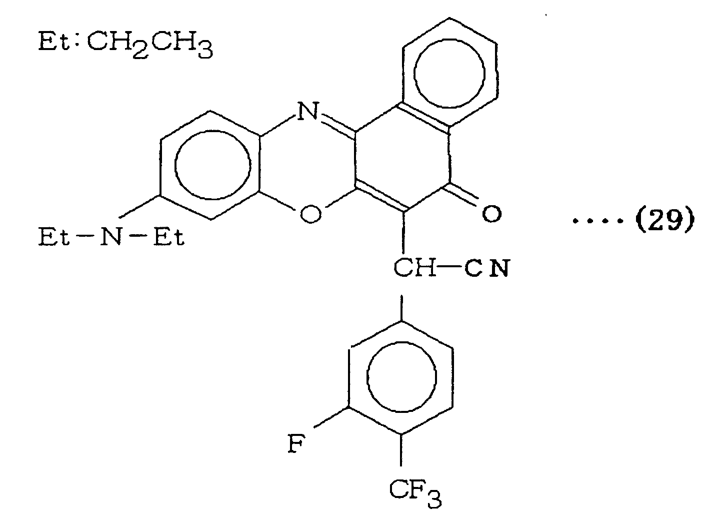

In a 5 ml graduated flask were placed 70.0 mg of polyvinyl carbazole, a product produced by Kanto Kagaku Co., Ltd., which polyvinyl carbazole will be abbreviated to PVK, 29.8 mg of BND, and 0.2 mg of Nile red luminescent compound G represented by formula (29). Dichloroethane was added to the mixture so that the total volume of the mixture was 5 ml. An EL element was prepared from the obtained solution containing this Nile red luminescent compound in the same way as in Working Example 9 above.

The highest luminance and the chromaticity of the EL element were measured with a Fast BM-7 measuring apparatus produced by TOPCON Corporation, with the voltage being raised gradually. When the voltage was 16 V and the current was 6.16 mA, the luminance was 1087 Cd/m² and the value on the x-axis in CIE chromaticity was 0. 6776. When the voltage was 17 V and the current was 9.32 mA, the luminance was 1444 Cd/m² and the value on the x-axis in CIE chromaticity was 0.6780. When the voltage was 18 V and the current was 12.71 mA, the luminance was 1622 Cd/m² and the value on the x-axis in CIE chromaticity was 0. 6787 . When the voltage was 19 V and the current was 15.73 mA, the luminance was 1455 Cd/m² and the value on the x-axis in CIE chromaticity was 0. 6790. When the voltage was 20 V and the current was 18.28 mA, the luminance was 1120 Cd/m² and the value on the x-axis in CIE chromaticity was 0.6710.

An ITO substrate was cleaned in the same manner as in Working Example 9 and the cleaned ITO substrate was set in a vacuum metallizer. Under a reduced pressure of not more than 1 x 10⁻⁶ torr, N,N'-diphenyl-N,N-di(m-tolyl)-benzidine (TPD) was deposited on the substrate, which results in a film having a thickness of 60 nm. Then, a mixture of tris (8-quinolinate) aluminum (Alq3) and Nile red luminescent compound G represented by formula (29), wherein the amount of the latter was 1. 7 weight %, was deposited on the surface of the TPD-applied substrate, so that the thickness of the upper film was 31 nm. Finally, the aluminum electrode of 150 nm in thickness was formed on the surface of the upper film by deposition. Thus an EL element was prepared.

The luminance and the chromaticity of the EL element were measured with the same method as in Working Example 9. When the voltage was 27 V and the current was 18.53 mA, the luminance was 6043 Cd/m², and the value on the x-axis in CIE chromaticity was 0.6326.

### (Working Example 22)

### <Synthesis of 6-amino-3-(diisopropylamino)phenol>

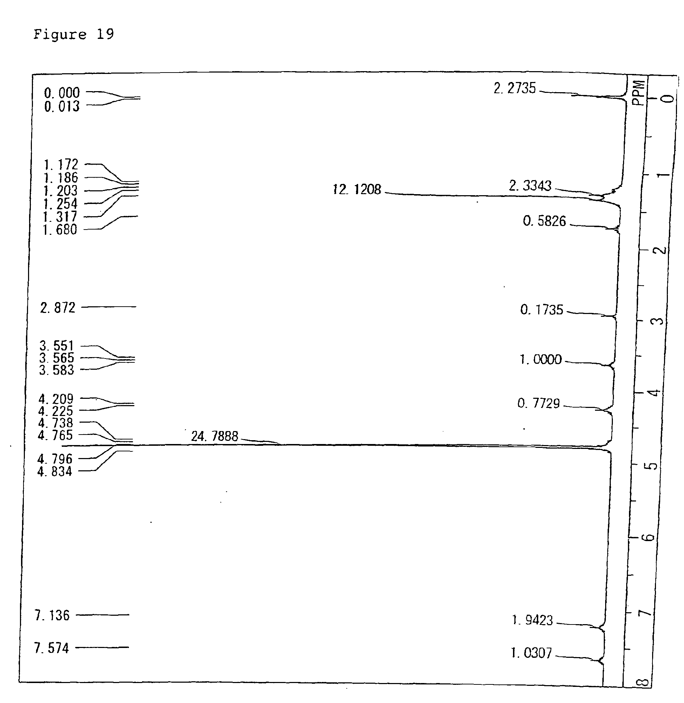

In a 500 ml three-necked flask, 26 g (115 mmol) of stannous chloride dihydrate and 28 ml of a concentrated hydrochloric acid were placed. The mixture was heated and boiled. 60 ml of an acetic acid solution of 5.5g (23.1 mmol) of 5-(diisopropylamino)-2-nitrophenol was dripped in the mixture. After the completion of the dripping, the obtained mixture was allowed to react at the reflux temperature for 1 hour. Then, the mixture was cooled to the room temperature. Water and acetic acid were completely distilled away. The residue was dissolved in 200 ml of water and the pH value of the aqueous solution was adjusted to 3-4 with a 5% aqueous solution of sodium hydroxide. The precipitated solids were filtered out and the filtrate was concentrated. The precipitated solids were washed with ether and vacuum-dried, which resulted in 16.9 g of a beige solid matter. This solid matter included salt. An NMR chart of this beige solid matter is shown in Figure 19. This solid was identified as 6-amino-3-(diisopropylamino)phenol.

### <Synthesis of a Nile Red Compound Derivative>

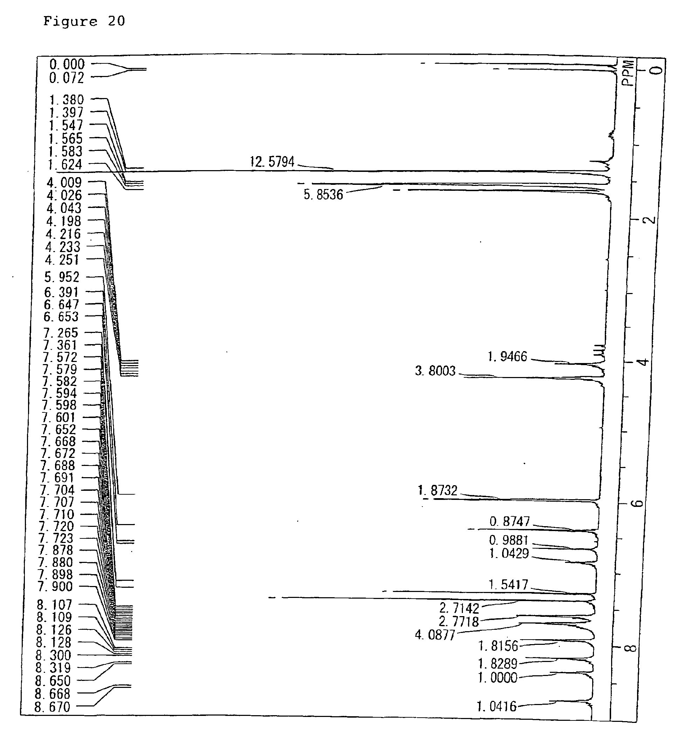

In a 500 ml pear-shaped flask were placed 16.9 g of 6-amino-3-(diisopropylamino)phenol, 4.0 g (23.1 mmol) of 2-hydroxy-1, 4-naphtoquinone, and 150 ml of ethanol. The mixture was allowed to react in the presence of boiling tips under reflux for 21 hours. The reaction liquid was concentrated with an evaporator, and alkalized with 200 ml of a 10% aqueous solution of sodium hydroxide. This alkaline liquid was extracted with chloroform and washed with water. The extract was dried with anhydrous sodium sulfate and then chloroform was distilled off. The treated extract was purified by a column chromatography employing silica gel. 0.50 g of the aimed Nile red compound derivative was obtained. An NMR chart of this Nile red compound derivative is shown in Figure 20.

### <Synthesis of a Nile Red Luminescent Compound>

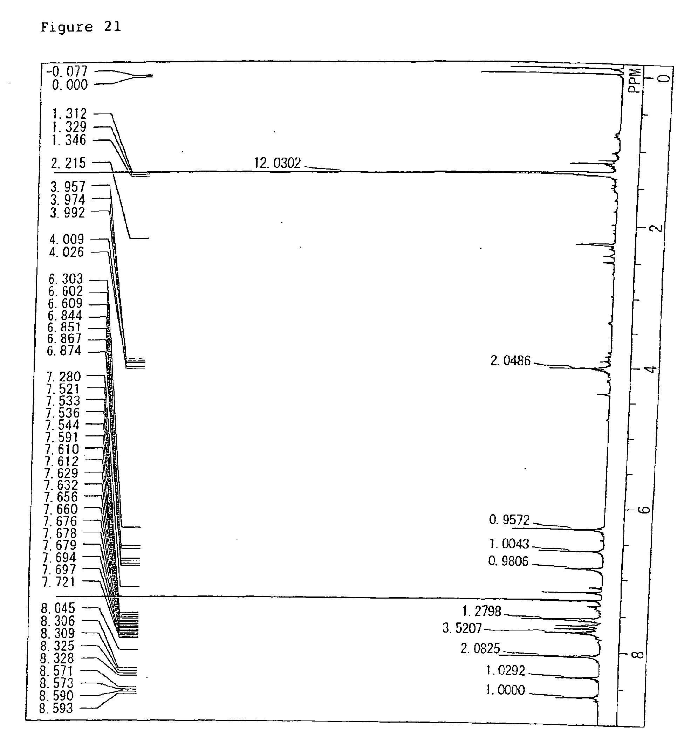

0.46 g (1.33 mmol) of the Nile red compound derivative, 0.50 g (1.99 mmol) of 3,5-bis(trifluoromethyl)phenylacetonitrile, and 50 ml of acetic anhydride were placed in a 100 ml pear-shaped flask. The solution in the pear-shaped flask was heated in a silicone oil bath to 135°C and allowed to react for 2 hours. Acetic anhydride was distilled off with an evaporator and the residue was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solids were purified by a column chromatography that used silica gel and benzene. 14 mg of a violaceous solid matter was obtained. The melting point of the violaceous solid matter was 183-185°C. An NMR chart of this product is shown in Figure 21. Based on these results, the obtained product was identified as the Nile red luminescent compound represented by formula (30).

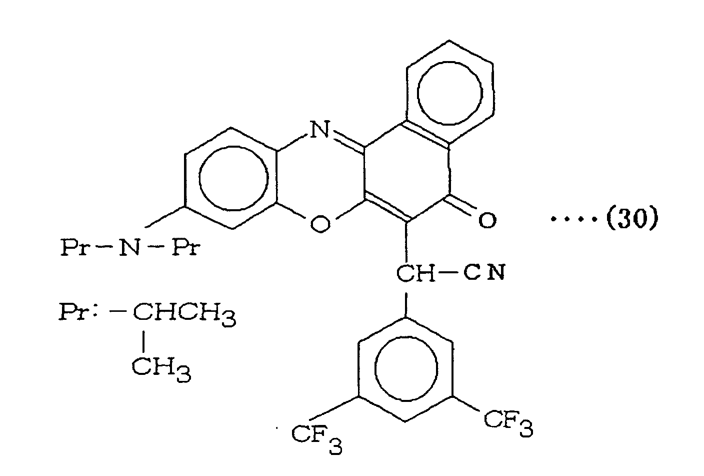

### (Working Example 23)

### <Synthesis of Ethylcarbazole Indophenol>

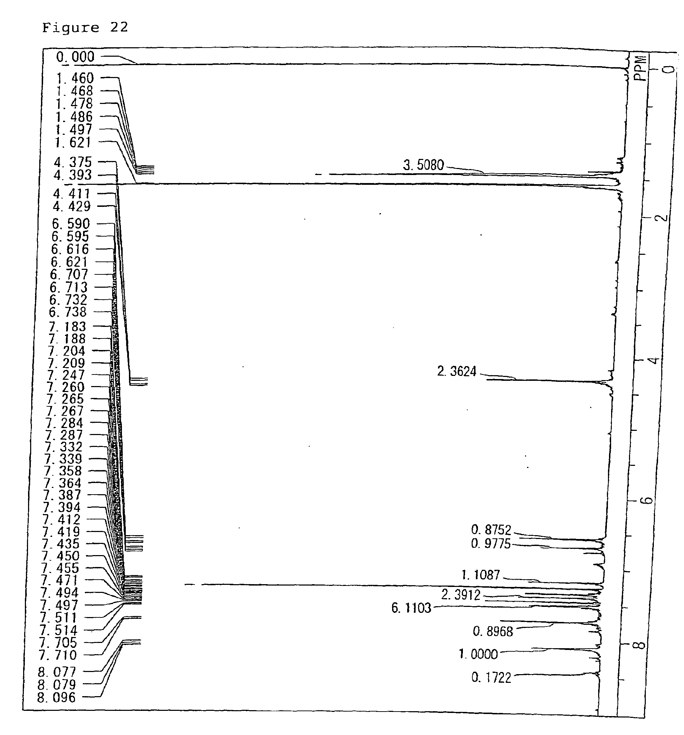

600 g of sulfuric acid was placed in a 1000 ml three-necked flask and the reaction system was cooled to -50 to -60°C. A mixture of 17.5 g (89.7 mmol) of ethylcarbazole and 25 g (122 mmol) of 4-nitrosophenol, which included 40% of water, was added gradually to the contents of the flask. Then, the reaction system was stirred for 6 hours, while being kept at temperatures of -30 to -40°C. After the completion of the reaction, the contents of the flask were introduced into ice water. While it was cold, the ice water with the contents of the flask was filtered with a suction funnel. The residue, which was a paste, was added to a 5% aqueous solution of sodium carbonate for neutralization. The solution was kept in a refrigerator for a night, and then it was filtered with a suction funnel. The residue, which was solids, were washed with water and dried in a desiccator including sulfuric acid. 36 g of a black solid matter was obtained. An NMR chart of the obtained compound is shown in Figure 22. From this NMR chart and the results of elemental analysis, the black solid matter was identified as the compound represented by formula (1a).

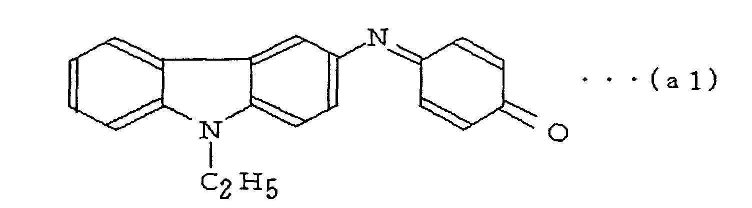

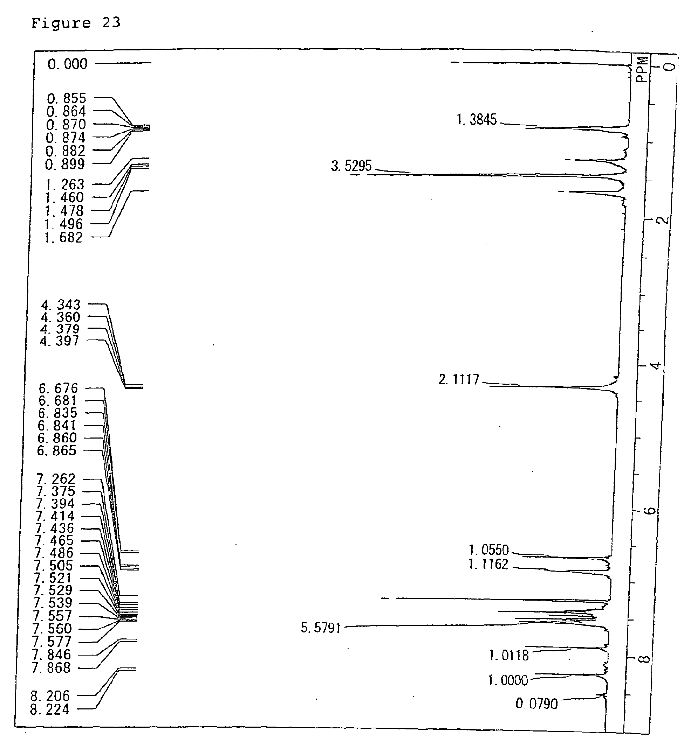

In a 200 ml three-necked flask were placed 10 g (33.3 mmol) of the compound (a1) obtained in the previous step, 2.13 g (66.6 mmol) of powdery sulfur, 0.4 g of iodine, and 50 ml of o-dichlorobenzene. The contents of the flask were heated to 190 °C in an oil bath and allowed to react for 2 hours. After the reaction, the contents were cooled to the room temperature and o-dichlorobenzene was distilled off. The residue was purified by a column chromatography that used chloroform for the developer, which resulted in 1.3 g of a black solid matter. An NMR chart of this product is shown in Figure 23. From this chart, this product was identified as the compound represented by formula (b1).

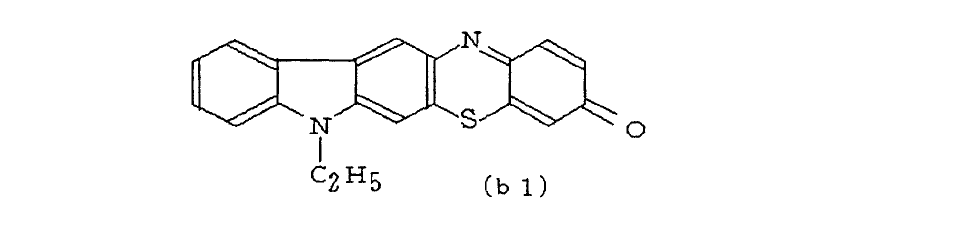

### (Working Example 24)

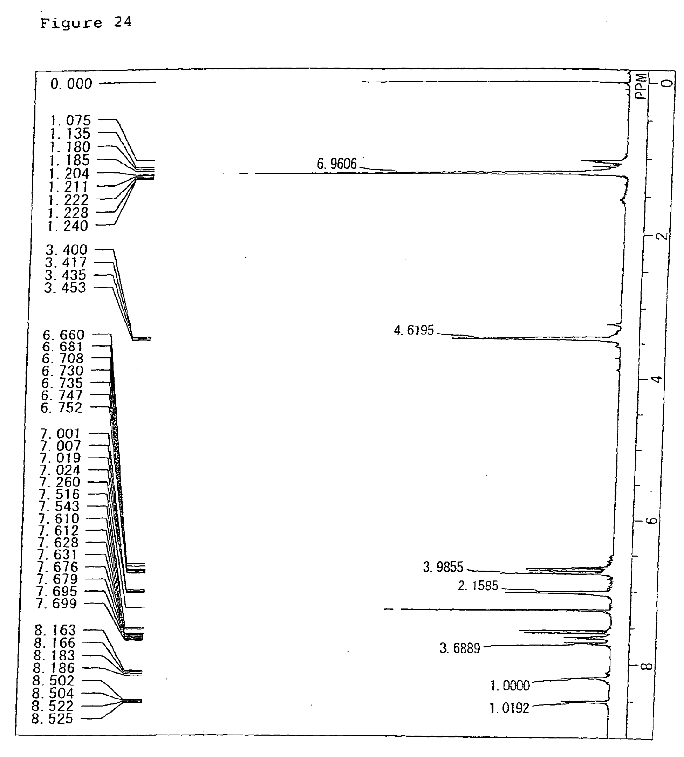

In a 2000 ml three-necked flask were placed 8.0 g (200 mmol) of sodium hydroxide and 300 g of water, and the obtained aqueous solution was cooled to 0°C. A solution made by dissolving 14.0 g (97.1 mmol) of 1-naphthol and 16.6 g (93.4 mmol) of N, N-diethyl-4-nitrosoaniline in 600 ml of ethanol was gradually dripped in the aqueous solution under the same temperature. The obtained mixture was stirred for 30 minutes, while being kept at the same temperature. Then, the temperature was raised to the room temperature and the mixture was allowed to react for 4 hours. After the reaction, the solution was concentrated to such an extent that the volume of the solution was reduced roughly to half. Then, the concentrated solution was diluted with 1500 ml of water, which led to the production of precipitates. The precipitates were washed with water, and dried. 8.0 g of a purplish red solid matter was obtained. An NMR chart of the solid matter is shown in Figure 24. From this chart, the obtained was identified as the compound represented by formula (c1).

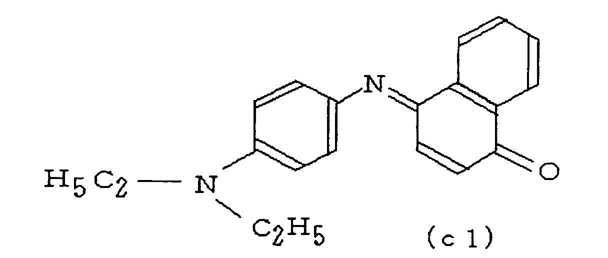

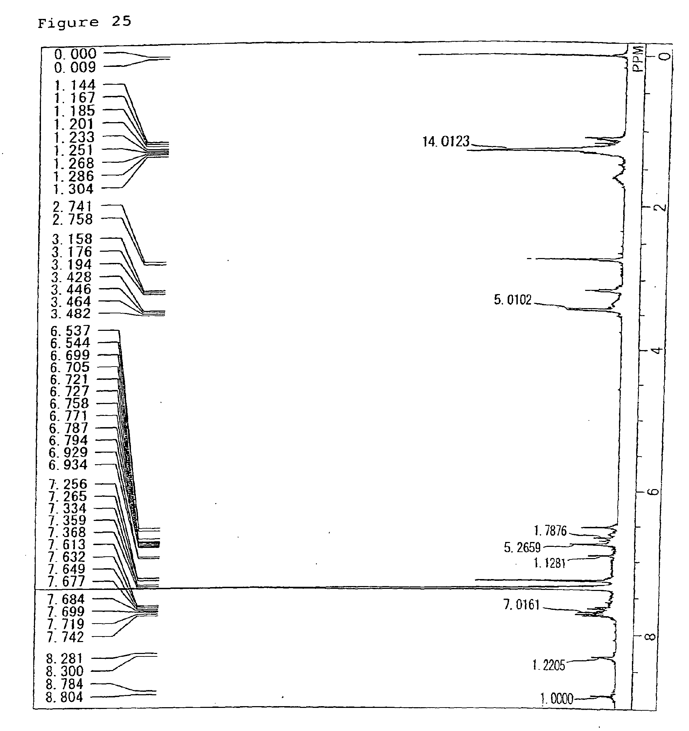

In a 300 ml pear-shaped flask were placed 8.0 g (26.3 mmol) of the compound (c1) obtained in the previous step, 1.69 g (52.6 mmol) of powdery sulfur, 0.3 g of iodine, and 50 ml of o-dichlorobenzene. The contents of the flask were heated to 185°C in an oil bath and allowed to react for 2.5 hours. After the reaction, the contents were cooled to the room temperature and o-dichlorobenzene was distilled off. The residue was purified by a column chromatography that used chloroform for the developer, which resulted in 1. 0 g of a black solid matter. An NMR chart of this product is shown in Figure 25. From this chart, this product was identified as the compound represented by formula (d1).

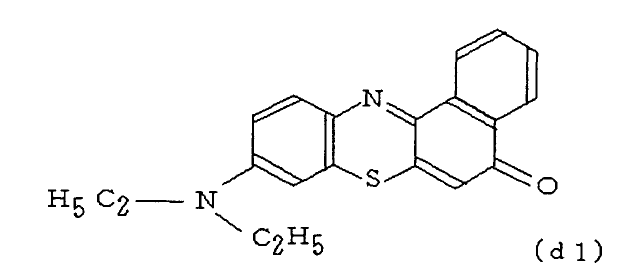

### (Working Example 25)

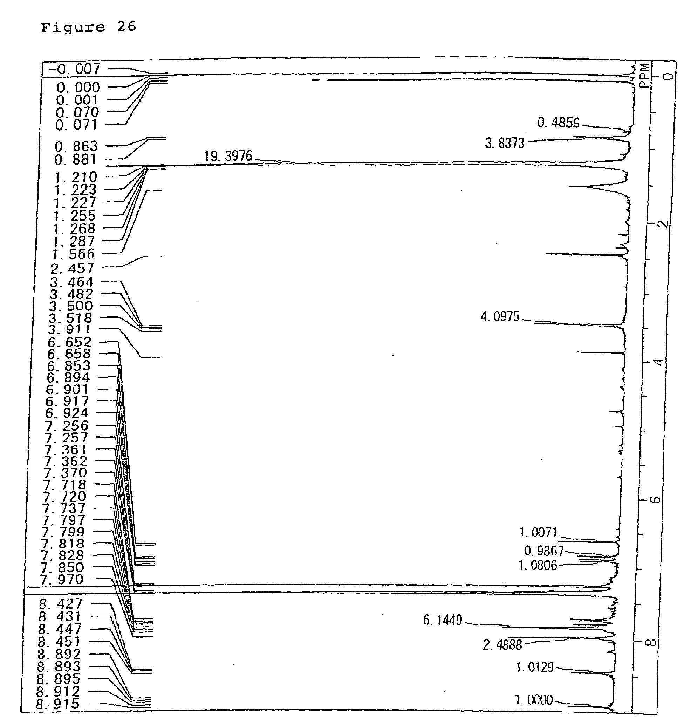

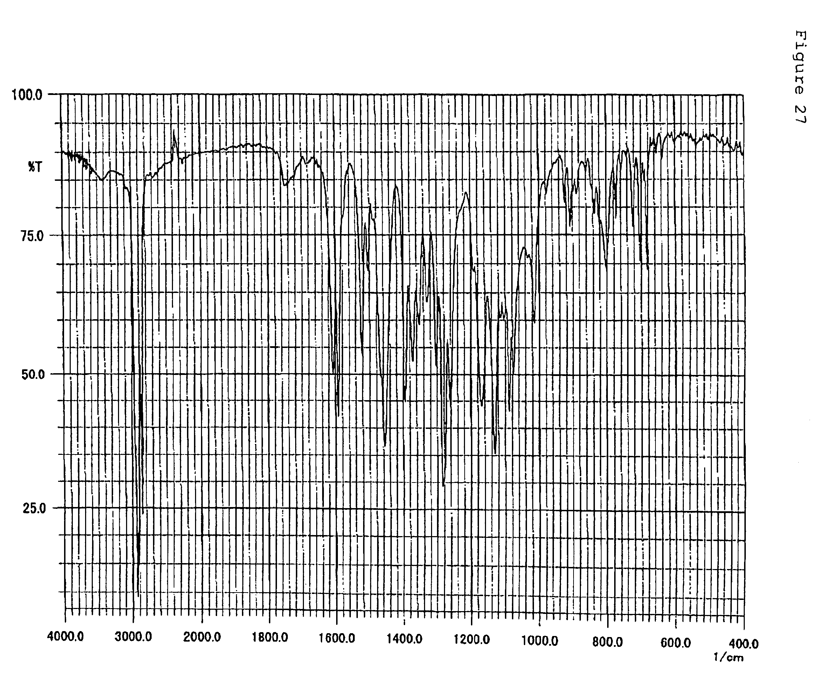

0.50g (1.57 mmol) of the Nile red luminescent compound obtained in Working Example 24, 0.40g (1.57 mmol) of 3,5-bis(trifluoromethyl)phenylacetonitrile, and 25 ml of acetic anhydride were placed in a 100 ml pear-shaped flask. The solution in the pear-shaped flask was heated in a silicone oil bath to 135°C and allowed to react for 4 hours. Acetic anhydride was distilled away with an evaporator and the remaining was dissolved in chloroform. This chloroform solution was washed with a 5% aqueous solution of sodium hydroxide and then with water. After the addition of sodium sulfate, the solution was allowed to stand for 30 minutes to be dried. The dried solution was concentrated with an evaporator. The obtained solid was purified by a column chromatography that used silica gel for the filler and benzene for the developer. 30 mg of violaceous solid was obtained in a 12% yield. A ¹H-NMR spectrum and an IR spectrum of this product are shown in Figures 26 and 27. Based on these results, the obtained product was identified as the chemical compound represented by formula (31).

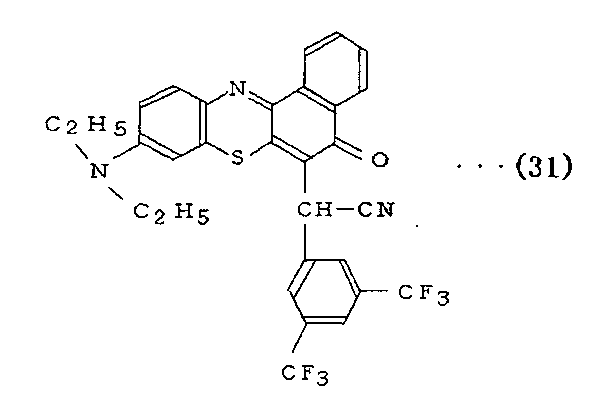

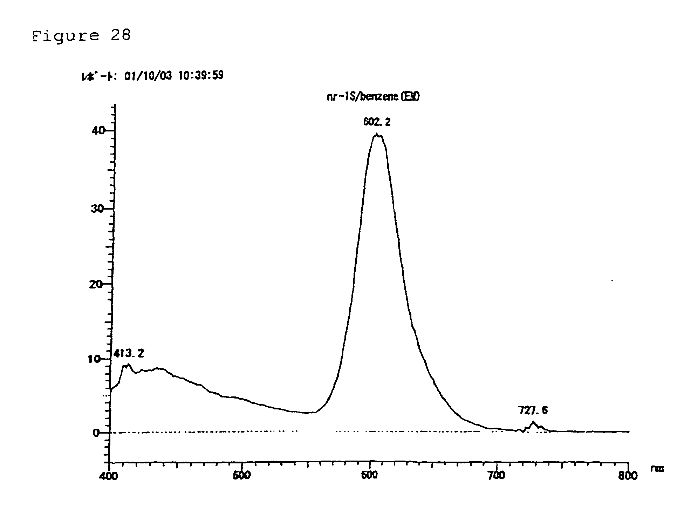

A sample solution was prepared by dissolving the Nile red luminescent compound represented by formula (31) in xylene, so that the concentration of the compound was 10 mg/L. This sample solution was loaded in a model F-4500 spectrofluorophotometer, a product by Shimadzu Corporation, and the fluorescence spectrum of the solution was measured under the following conditions. The measured spectrum is shown in Figure 28.

### Conditions of Measurement

Measuring mode: Wavelength scanning
Exciting wavelength: 365 nm
Wavelength at which the emission of fluorescence started: 400 nm
Wavelength at which the emission of fluorescence ended: 800 nm
Scanning speed: 1200 nm/min.
Slit on the side of excitation: 5.0 nm
Slit on the side of fluorescence emission: 5.0 nm
Photomal voltage: 700 V

Figure 28 confirms that the fluorescence of the Nile red luminescent compound obtained in this example emitted red light.

### (Working Example 26)

### <Synthesis of a Nile Red Luminescent Compound>

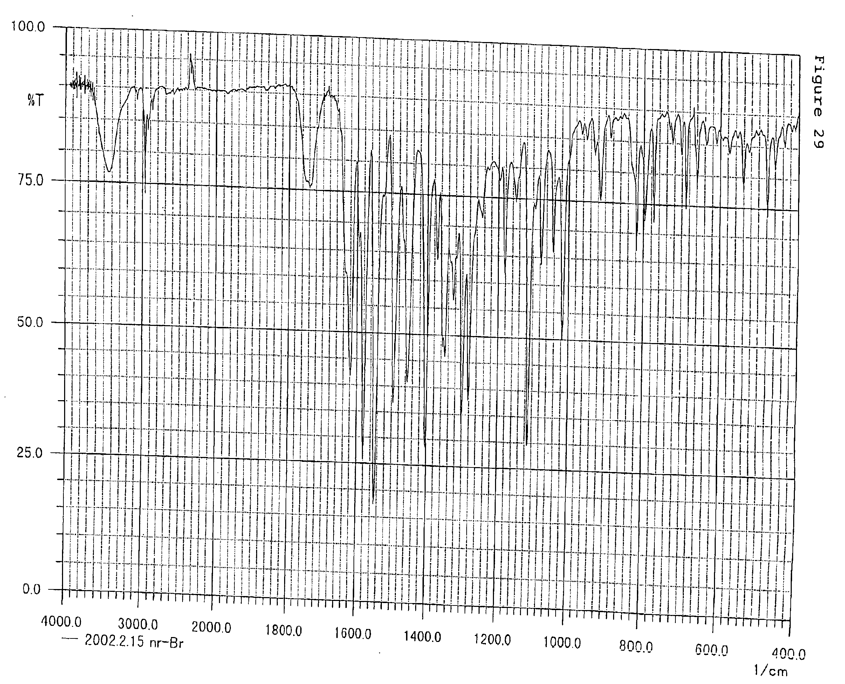

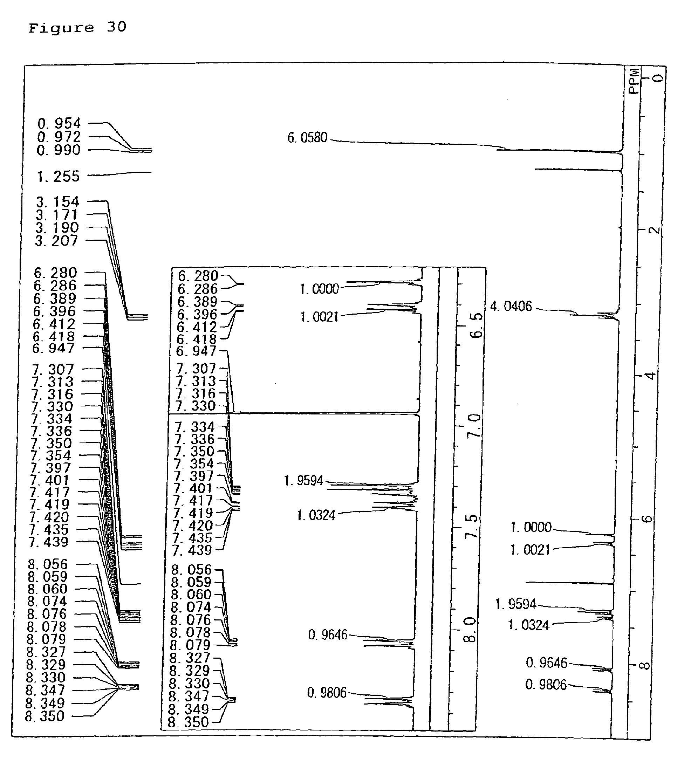

10.0 g (31.4 mmol) of Nile red, 6.20 g (34.8 mmol) of N-bromosuccinimide, 0.20 g of AIBN, and 780 ml of carbon tetrachloride were placed in a 2000 ml pear-shaped flask. The solution in the pear-shaped flask was heated in a silicone oil bath to 100°C and allowed to react for 2 hours. Carbon tetrachloride was distilled away with an evaporator and the remaining solids were dissolved in 700ml of chloroform. Solids were obtained. The solids were purified by a column chromatography that used chloroform for the developer. The purified was further recrystallized in toluene. 5.1 g of a deep green solid matter was obtained. An IR spectrum of this solid matter is shown in Figure 29 and an NMR chart thereof is shown in Figure 30.

Based on these results, the obtained product was identified as a Nile red luminescent compound emitting red light that has the structure represented by formula (18).

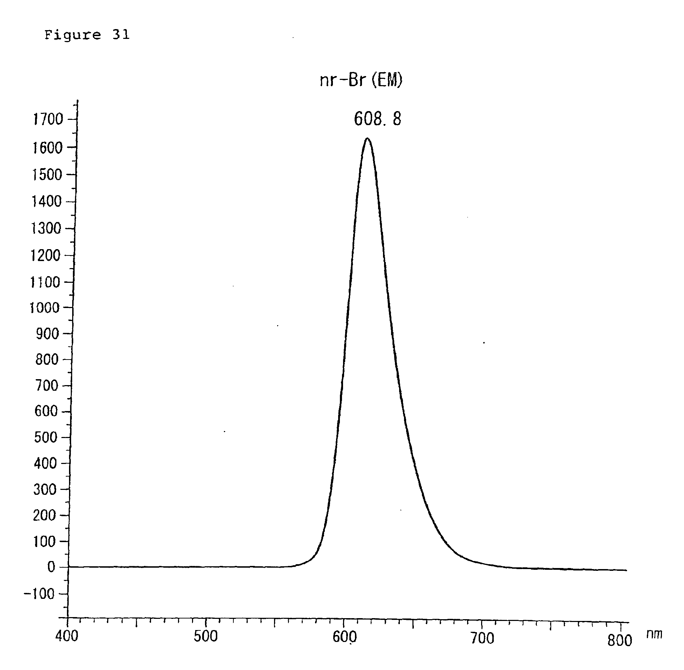

A fluorescence spectrum of this product is shown in Figure 31.

### Industrial Applicability

This invention can provide a novel Nile red luminescent compound capable of emitting at a high luminance a light that has a peak wavelength of which color is very closer crimson, and of enduring heat and light. Conventional technologies could not realize such luminescent compounds.

This invention can also provide a novel Nile red luminescent compound, from which a luminescence element emitting white light can be prepared.

Furthermore, this invention can provide an industrial process for easily producing the Nile red luminescent compound.

Still further, this invention can provide an EL element, of which light-emitting layer contains the novel Nile red luminescent compound, capable of emitting a crimson light at a high luminance. Also, when the light-emitting layer includes a green light-emitting compound and a blue light-emitting compound together with the Nile red luminescent compound, a luminescence element emitting white light can be provided.

## Claims

1. A Nile red luminescent compound emitting red light that has a structure represented by formula (1): 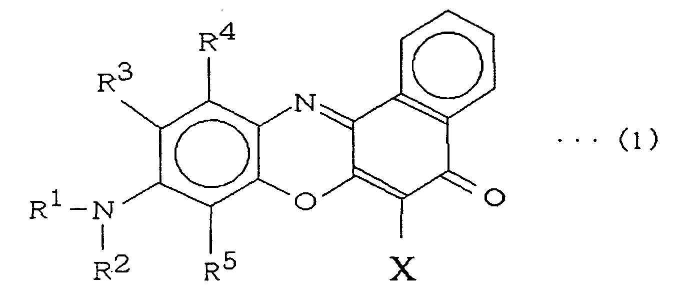 wherein R¹ is hydrogen atom or an alkyl group, or forms -CH₂CH₂-CR⁶R⁷- together with R³ (wherein the carbon atom of -CR⁶R⁷- moiety is bound to the benzene moiety of the formula (1), each of R⁶ and R⁷ is hydrogen atom or an alkyl group, and R⁶ and R⁷ may be the same or different from each other); R² is hydrogen atom or an alkyl group, or forms -CH₂CH₂-CR⁸R⁹- together with R⁵ (wherein the carbon atom of -CR⁸R⁹- moiety is bound to the benzene moiety of the formula (1), each of R⁸ and R⁹ is hydrogen atom or an alkyl group, and R⁸ and R⁹ may be the same or different from each other); R³ is hydrogen atom, forms -CH₂CH₂-CR⁶R⁷- with R¹, or forms with R⁴ a naphthalene ring including as a part thereof the benzene moiety of the formula (1); R⁴ is hydrogen atom, or forms with R³ a naphthalene ring including as a part thereof the benzene moiety of the formula (1); R⁵ is hydrogen atom, or forms -CH₂CH₂-CR⁸R⁹- with R²; and X is a halogen atom.

2. A process of producing the Nile red luminescent compound emitting red light represented by the formula (1), comprising reacting with a halogenating agent a Nile red pigment represented by general formula (2): 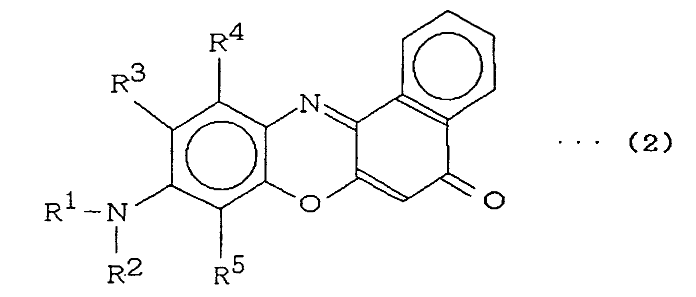 wherein R¹, R², R³, R⁴ and R⁵ are respectively the same as those defined in claim 1.

3. A Nile red compound emitting red light that has a structure represented by formula (3). 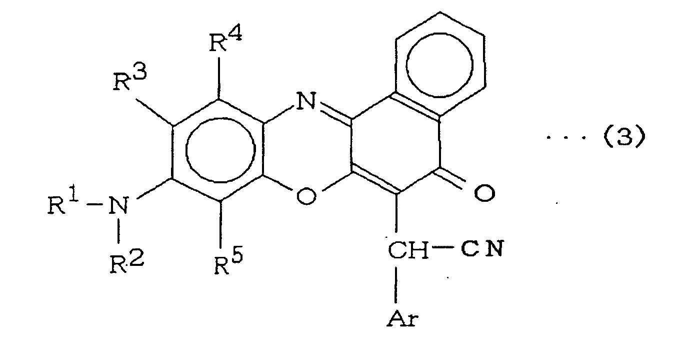 wherein R¹, R², R³, R⁴ and R⁵ mean the same atoms and groups as those defined in claim 1; Ar means one of formulae (4), (6) and (7) : 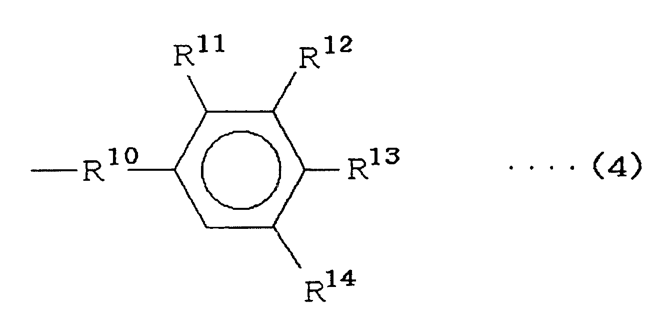 wherein R¹⁰ is a single chemical bond or methylene group; R¹¹ is hydrogen atom, or forms -CF₂-O-CF₂- with R¹²; R¹² is fluorine atom, cyano group or a lower alkyl having 1-5 carbon atoms and at least one fluorine atom, forms -CF₂-O-CF₂- with R¹¹, or forms -CF₂-O-CF₂- with R¹³; R¹³ is hydrogen atom, cyano group, fluorine atom or a lower alkyl having 1-5 carbon atoms and at least one fluorine atom, forms -CF₂-O-CF₂- with R¹², or is a group represented by formula (5); and R¹⁴ is hydrogen atom or a lower alkyl having 1-5 carbon atoms and at least one fluorine atom when R¹³ is hydrogen atom, and R¹⁴ is hydrogen atom when R¹³ is not hydrogen atom, 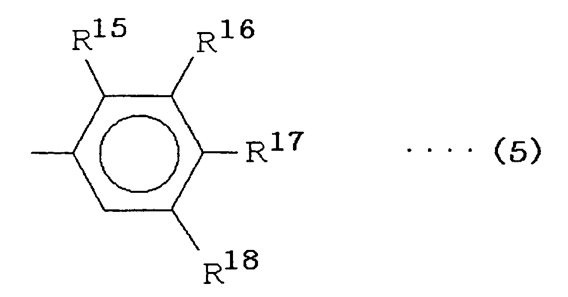 wherein R¹⁵ is hydrogen atom, or forms -CF₂-O-CF₂- with R¹⁶; R¹⁶ is fluorine atom, cyano group or a lower alkyl having 1-5 carbon atoms and at least one fluorine atom, forms -CF₂-O-CF₂- with R¹⁵, or forms -CF₂-O-CF₂- with R¹⁷; R¹⁷ is hydrogen atom, cyano group, fluorine atom or a lower alkyl having 1-5 carbon atoms and at least one fluorine atom, or forms -CF₂-O-CF₂- with R¹⁶; and R¹⁸ is hydrogen atom or a lower alkyl having 1-5 carbon atoms and at least one fluorine atom when R¹⁷ is hydrogen atom, and R¹⁸ is hydrogen atom when R¹⁷ is not hydrogen atom, 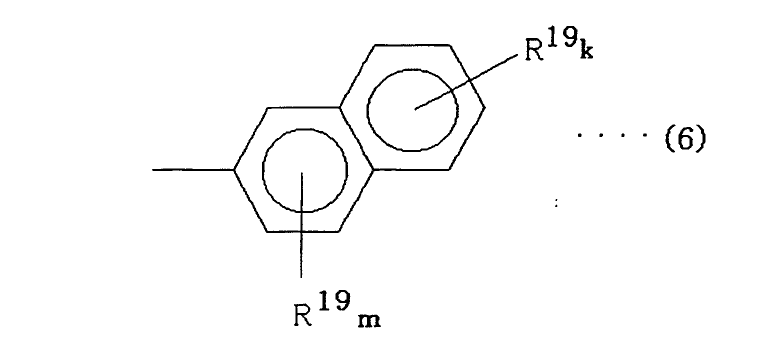 wherein R¹⁹ is fluorine atom, cyano group, or a lower alkyl having 1-5 carbon atoms and at least one fluorine atom; k is an integer of 1-4, m is an integer of 1-3, and all of the R¹⁹ groups may be the same or different from each other, 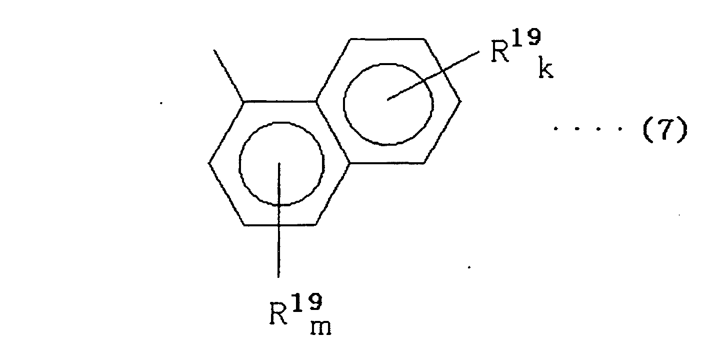 wherein R¹⁹ is fluorine atom, cyano group, or a lower alkyl having 1-5 carbon atoms and at least one fluorine atom; k is an integer of 1-4, m is an integer of 1-3, and all of the R¹⁹ groups may be the same or different from each other.

4. A process of preparing the Nile red luminescent compound emitting red light represented by the formula (3) comprises reacting the Nile red pigment compound represented by the formula (2) with an electron attractive aromatic acetonitrile represented by formula (8): 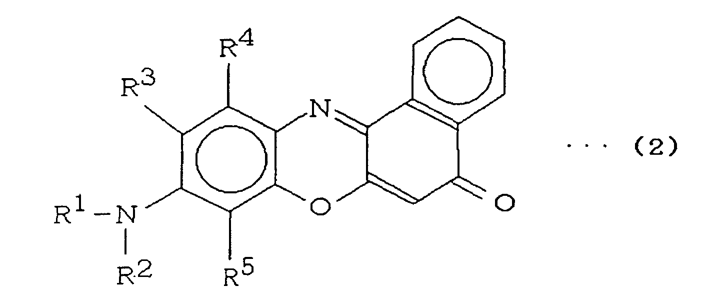 wherein R¹, R², R³, R⁴ and R⁵ are respectively the same as those defined in claim 1,
NC-CH₂-Ar (8)
wherein Ar is the same as that defined in claim 3.

5. A Nile red luminescent compound emitting red light that has a structure represented by formula (9): 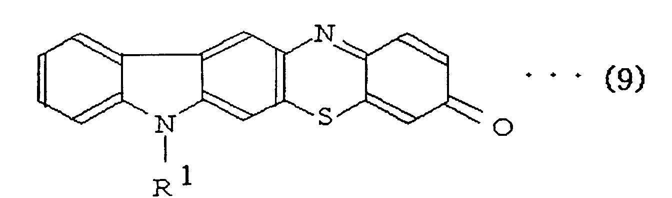 wherein R¹ is hydrogen atom or an alkyl group.

6. A Nile red luminescent compound emitting red light that has a structure represented by formula (10): 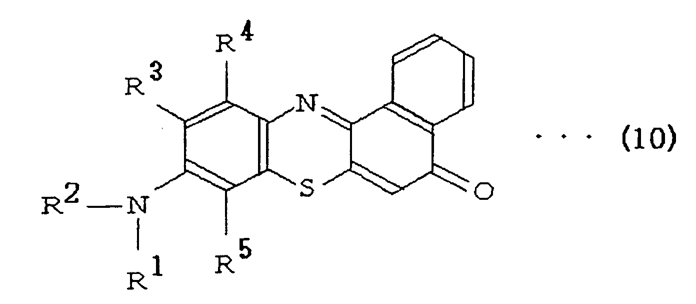 wherein R¹, R², R³, R⁴ and R⁵ are respectively the same as those defined in claim 1.

7. The process of producing the Nile red luminescent compound according to claim 5 comprising reacting 4-nitrosophenol with a carbazole, the nitrogen atom of which is bonded with a substituent R¹, wherein R¹ is hydrogen atom or an alkyl group, to produce an intermediate compound, and reacting the intermediate compound with sulfur.

8. A process of producing the Nile red luminescent compound according to claim 6 comprising reacting 1-naphphol with a 4-nitrosoaniline, the amino group of which is bonded with substituents R¹ and R², wherein each of R¹ and R² is hydrogen atom or an alkyl group, and R¹ and R² may be the same or different from each other, to produce an intermediate; and reacting the intermediate with sulfur.

9. A Nile red luminescent compound emitting red light that has a structure represented by formula (11) : 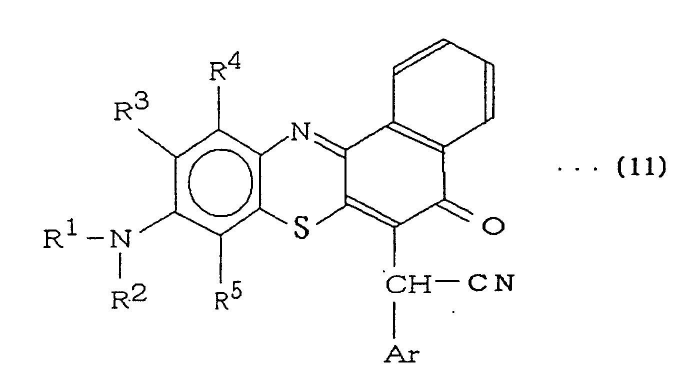 wherein R¹, R², R³, R⁴ and R⁵ are respectively the same as those defined in claim 1, and Ar is the same as that defined in the claim 3.

10. A process of producing the Nile red luminescent compound emitting red light according to claim 9 comprising reacting the Nile red luminescent compound emitting red light represented by the formula (10) with an electron attractive aromatic acetonitrile represented by the formula NC-CH₂-Ar, wherein Ar is the same as that defined in claim 3.

11. A luminescence element comprising a pair of electrodes and a light-emitting layer including at least one of the Nile red luminescent compounds as claimed in any one of claims 1, 3, 5, 6 and 9.

12. A luminescence element as claimed in claim 11, further comprising a hole-transporting layer between the light-emitting layer and a cathode, which is one of the electrodes.

13. The luminescence element as claimed in claim 12, wherein the light-emitting layer includes at least one of the Nile red luminescent compounds as claimed in any one of claims 1, 3, 5, 6 and 9, and a host pigment.

14. The luminescence element as claimed in claim 12 or 13, wherein the light-emitting layer and the hole-transporting layer are formed by deposition.

15. The luminescence element as claimed in claim 11, wherein the light-emitting layer includes at least one of the Nile red luminescent compounds as claimed in any one of claims 1, 3, 5, 6 and 9, an electron-transporting substance, and a hole-transporting high polymer.

16. The luminescence element as claimed in claim 15, wherein the light-emitting layer is formed through the application of the layer.
